(19) Europäisches Patentamt / European Patent Office / Office européen des brevets

(11) **EP 2 660 746 A1**

(12) **EUROPEAN PATENT APPLICATION**

(43) Date of publication:
06.11.2013 Bulletin 2013/45

(51) Int Cl.:
*G06F 19/18* (2011.01)       *G06F 19/12* (2011.01)

(21) Application number: **12166187.0**

(22) Date of filing: **30.04.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(71) Applicant: **Royal College of Surgeons in Ireland Dublin 2 (IE)** | (72) Inventors:<br>• **Prehn, Jochen**<br>　**Dublin 14 (IE)**<br>• **Lindner, Andreas**<br>　**Dublin 8 (IE)**<br>• **Huber, Heinrich**<br>　**Sandyford, Dublin 18 (IE)**<br><br>(74) Representative: **Purdylucey Intellectual Property 6-7 Harcourt Terrace Dublin 2 (IE)** |

(54) **Dose-response medical outcome model predictor system and method**

(57) A computer-implemented method for predicting quantitatively whether an adjuvant or neoadjuvant chemotherapeutic is successful in treating an individual suffering from cancer, the method comprising the steps of assaying a cancerous biological sample and a normal biological sample from the individual to determine the concentration of two or more BCL-2 family members in each sample; inputting the concentration value for at least two BCL-2 family members of each sample into a non-linear protein-protein network computational model and adapted to calculate quantitative protein profiles over time from quantitative molecular interaction data and assess the mitochondrial outer membrane permeabilisation apoptosis pathway invoked by chemotherapeutically-induced stress; and processing said concentration values using said computational model to determine the interaction of pro-apoptotic and pro-survival BCL-2 family members invoked after chemotherapeutic-induced stress in the sample; and outputting a quantitative prediction value of the likelihood of treatment success using the adjuvant or neoadjuvant chemotherapeutic treatment.

Figure 1

EP 2 660 746 A1

**Description**

**Field of the Invention**

The invention relates to a computer-implemented system and method for predicting a patient's response to a treatment. In particular the invention relates to a quantitative method and system for predicting a patient's response to cancer treatment and calculates effective treatment dosages.

**Background to the Invention**

Cancer treatments are ineffective with poor overall 5 year survival rates of just 55% for all cancers. Treatment regimes that do not improve the patient's health or mitigate patient burden pose significant and unnecessary costs to the health care sector that could have been directed to more efficient therapies. The decision for the treatment that has the best success rate in individual patients and minimizes patient risk and burden is therefore decisive. To decide on the best individually suitable treatment, reliable clinical tools are needed.

Chemotherapeutic treatment shall kill cancer cells while preserving healthy tissue. Finding the right treatment dose is therefore decisive. The dose where cancer cells are killed and healthy tissue is preserved influences the therapeutic window. The size of this therapeutic window (and whether or not there is any) depends on the cancer type, cancer stage and patient individual characteristics.

Cells translate intrinsic and extrinsic stress conditions into an internal biochemical cascade that acts as a regulator between protective and cell death pathways. Deregulation of these processes leads to an imbalance of tissue homeostasis, hypersensitivity or to the proliferation of malignant cells. Mitochondrial outer membrane permeabilisation (MOMP) is regarded as the point of no return in apoptosis, yet the exact mechanism of how this stress integration and survival balance is executed by different BCL2-family member proteins remains unclear. It is broadly accepted that the BCL2-family member proteins BAK and BAX, also called effectors, homo-oligomerise to form pores in the mitochondrial membrane in a redundant fashion and that their functionality is regulated by the interplay of other pro-apoptotic and anti-apoptotic BCL2-family members. It is currently controversial whether or not an independent activation step of BAK and BAX by some BH3-only proteins such as BID, BIM and PUMA is required (as assumed by the 'direct activation model' in contrast to the 'indirect activation model').

Mathematical modelling allows assembling existing knowledge into a holistic model. This facilitates the establishment and *in-silico* tests of new hypotheses before model predictions are eventually validated in wet lab experiments. Using tools from control theory, they may supplement quantitative data that otherwise cannot be determined experimentally. State of the art models put emphasis on pre-MOMP or post-MOMP processes or try to establish a holistic model of extrinsic or intrinsic stress or apoptosis. As a recent example, Zhang and co-workers (T. Zhang, P. Brazhnik, and J. J. Tyson, 'Computational analysis of dynamical responses to the intrinsic pathway of programmed cell death', Biophys J, vol. 97, p. 415-34, 2009) established a comprehensive model of how intrinsic and extrinsic stress proceeds through the apoptotic cascade. Their approach consisted of apoptosis initiation, amplification and execution modules that together guarantee cellular robustness versus sub-threshold stimuli. Similar to their approach, Chu *et al* (L. H. Chu and B.S. Chen, BMC Systems Biology 2008 2:56) provided a theoretical study of how intrinsic and extrinsic stress translates into a protein-interaction network in cancer cells that governs the execution of apoptosis.

Systems and models that shall be applied to the investigation of desired or unintentional apoptotic effects of drug compounds, to individualized cancer therapies, or similar clinical settings, consequently need to include above intricacies. However, none of the practicing techniques have been proven to be suitable for predicting cancer cell death in response to state of the art chemotherapeutics and no such approach can be used for dosage decisions to induce cancer cell death or to preserve healthy cells in different tumour or non-malignant tissues.

It is an object of the present invention to overcome at least one of the above-mentioned problems.

**Summary of the Invention**

According to the present invention there is provided, as set out in the appended claims, a computer-implemented method for predicting quantitatively whether an adjuvant or neoadjuvant chemotherapeutic treatment will be or is being successful in treating an individual suffering from cancer, the method comprising the steps of:

assaying a cancerous biological sample and a normal biological sample from the individual to determine the concentration of two or more BCL-2 family members in each sample;
inputting the concentration value for at least two BCL-2 family members of each sample into a non-linear protein-protein network computational model and adapted to calculate quantitative protein profiles over time from quantitative molecular interaction data and assess the mitochondrial outer membrane permeabilisation apoptosis pathway invoked by chemotherapeutically-induced stress;

processing said concentration values using said computational model to determine the interaction of pro-apoptotic and pro-survival BCL-2 family members invoked after chemotherapeutic-induced stress in the sample; and

outputting a quantitative prediction value of the likelihood of treatment success using the adjuvant or neoadjuvant chemotherapeutic treatment.

In one embodiment of the present invention, the method comprises the further step of outputting a quantitative, dose-dependent prediction value of the likelihood of treatment success using novel apoptosis sensitizers.

In one embodiment of the present invention, the method further comprises the step of estimating a minimal quantitative dose value of a chemotherapeutic required to induce the mitochondrial outer membrane permeabilisation apoptosis pathway in the sample, such that any cancer cell is killed while preserving healthy cells of the individual suffering from cancer.

In one embodiment of the present invention, the quantitative minimal dose is estimated based on the concentration values of the BCL-2 family members required to induce the process of mitochondrial outer membrane permeabilisation in the cancer cell.

The invention has established a comprehensive model that is applicable to individual stress situations and protein expression levels. This has allowed investigations of how the combination of BH-3 only family members and their mimetics are apoptotically processed using the well-characterized specificity between certain pro-apoptotic BCL2 members and their anti-apoptotic counterparts. As BAK is localized to the mitochondrial membrane and bound to voltage dependent ion channels (VDAC2) while inactive and cytosolic BAX translocates upon stress to mitochondria, separate state variables are introduced that allow investigation of their separate contributions to MOMP.

One of the problems with the Zhang *et al.* and the Chu *et al* models, which the present invention overcomes, is that they did not focus on the specific regulation of BH-3 only anti-apoptotic proteins. Furthermore, these models simplified BAK and BAX entities into a common state variable. Likewise, different tissues or tumour cell lines show specific expression profiles of individual anti-apoptotic BCL2 family members. In particular, neither approach took into account the interactions between specific BH3-only proteins and specific pro-survival BCL-2 proteins and their actual kinetics of interaction. Therefore, neither of these approaches, or any other approach prior to or after the Zhang and Chu studies, were able to investigate how a patient characteristic mix of the expression levels of different specific BH3-only and specific pro-survival BCL-2 proteins translates a chemotherapeutic stimulus into cell death. By similar means, no current approach is able to correctly mimic a specific and dose-dependent chemotherapeutic stimulus as such stimuli are known to induce a specific mix of different BH3-only proteins.

In one embodiment of the present invention, the concentration values inputted into the computational model are processed to provide an apoptosis status, which is correlated with a known response to provide said quantitative, dose-dependent prediction value to predict the individual's response to treatment and/or the minimal dose necessary to kill cancer cells.

In one embodiment of the present invention, said processing step calculates an activation profile over time of pro-apoptotic effector BCL-2 family member proteins and compares the result with known results to quantitatively predict the individual's response to treatment and/or the minimal dose necessary to kill cancer cells.

In one embodiment of the present invention, the BCL-2 family members are selected from BAK, BAX, BCL-2, BCL-(X)L, BCL-(X)S, BCL-W, MCL-1, BIM, BID, BAD, PUMA and NOXA.

In one embodiment of the present invention, the pro-apoptotic effector BCL-2 proteins are BAK and BAX.

In one embodiment of the present invention, the concentration value for each of the at least two BCL-2 family member is representative of protein levels for the sample.

Absolute protein levels can be obtained from biopsies, resected tumor material, or formalin-fixed, paraffin-embedded histopathology material using reverse phase protein arrays, quantitative Western Blotting, tissue microarray immunostaining or immunohistochemistry. This protein data will feed into the computational model that analyses protein-protein interaction.

Generally speaking, the biological sample is a blood sample, especially blood serum or plasma. However, other biological samples may also be employed, for example, cerebrospinal fluid, saliva, urine, lymphatic fluid, or cell or tissue extracts or biopsy or tissue biopsy.

Generally speaking, the individual is a human, although the computer-implemented method of the invention is applicable to other higher mammals.

In this specification, the term "cancer" should be understood to mean a cancer that is treated by chemotherapeutic regimens. An example of such a cancer include multiple myeloma, prostate cancer, glioblastoma, lymphoma, fibrosarcoma; myxosarcoma; liposarcoma; chondrosarcom; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; squamous cell carcinoma; basal cell carcinoma; adenocarcinoma; sweat gland carcinoma; sebaceous gland carcinoma; papillary carcinoma;

papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias.

In a further embodiment of the present invention, there is provided a computer-implemented system for quantitatively predicting whether an adjuvant or neoadjuvant chemotherapeutic treatment will be or is being successful in treating an individual suffering from cancer, the system comprising:

means for assaying a cancerous biological sample and a normal biological sample from the individual to determine the concentration of two or more BCL-2 family members in each sample;
means for inputting the concentration value for at least two BCL-2 family members of each sample into a non-linear protein-protein network computational model and adapted to calculate quantitative protein profiles over time from quantitative molecular interaction data and assesses the mitochondrial outer membrane permeabilisation apoptosis pathway invoked by chemotherapeutically-induced stress;
means for processing said concentration values using said computational model to quantitatively determine the interaction of pro-apoptotic and pro-survival BCL-2 family members invoked after chemotherapeutic-induced stress in the sample; and
means for outputting a quantitative prediction value of the likelihood of treatment success using the adjuvant or neoadjuvant chemotherapeutic treatment.

In one embodiment of the present invention, said processing step calculates an activation profile over time of pro-apoptotic effector BCL-2 family member proteins and compares the result with known results to predict the individual's response to treatment and/or the minimal dose necessary to kill cancer cells.

In one embodiment of the present invention, the BCL-2 family members are selected from BAK, BAX, BCL-2, BCL-(X)L, BCL-(X)S, BCL-W, A1, MCL-1, BIM, BID, BAD, PUMA and NOXA.

In the specification, the term "positive outcome" should be understood to mean a patient who responds positively to chemotherapeutic treatment, while the term "negative outcome" should be understood to mean a patient who does not respond to chemotherapeutic treatment.

In the specification, there term "treatment" should be understood to mean standard chemotherapeutic treatment, novel co-treatment regimes and/or novel adjuvant treatments (including non-standard/experimental treatments). Chemotherapeutic treatments are those using compounds such as for example chemotherapeutic agents fluorouracil (5FU), oxaliplatin, irinotecan, etoposide, cisplatin, doxorubicin, and receptor kinase inhibitors such as cetuximab and sorafenib. As an example, conventional treatment for stage 2 (non metastatic) colorectal cancer is surgical resection with optional chemotherapy based on 5FU/oxaliplatin and leucovorin (FOLFOX) or 5FU/ironotecan (FOLFIRI). These drugs exert their beneficial activities by inducing DNA damage ('genotoxic drugs'). For stage 3 and stage 4 colorectal cancer FOLFOX or FOLFIRI chemotherapeutic treatment is applied in the presence or absence of the angiogenesis inhibitor bevacizumab (Avastin) or the HER2 inhibitor Cetuximab (kinase inhibition). Novel adjuvant, non-standard and experimental treatments include BH3-only mimetics Abbot's Navitoclax and ABT-737, Gemin X's Obatoclax, Ascenta's AT-101, and Gossypol.

In the specification, the term "pro-apoptotic biomarker" should be understood to mean a molecule involved in promoting and/or progressing the process of programmed cell death (biochemical events leading to characteristic cell changes including blebbing, cell shrinkage, nuclear fragmentation, chromatin condensation, and chromosomal DNA fragmentation) and cell death by modifying the so-called effector proteins (Bak and Bax) from an inactive to an active form. Pro-apoptotic biomarkers can be selected from the group comprising BIM, BID, BAD, PUMA, NOXA, and BCL-(X)S.

In the specification, the term "BCL2 anti-apoptotic proteins" should be understood to mean a molecule involved in preventing and/or stopping the process of programmed cell death selected from the group comprising BCL-2, BCL-(X) L, BCL-W, A1 and MCL-1.

There is also provided a computer program comprising program instructions for causing a computer program to carry out the above method which may be embodied on a record medium, carrier signal or read-only memory.

## Brief Description of the Drawings

The invention will be more clearly understood from the following description of an embodiment thereof, given by way of example only, with reference to the accompanying drawings, in which:-

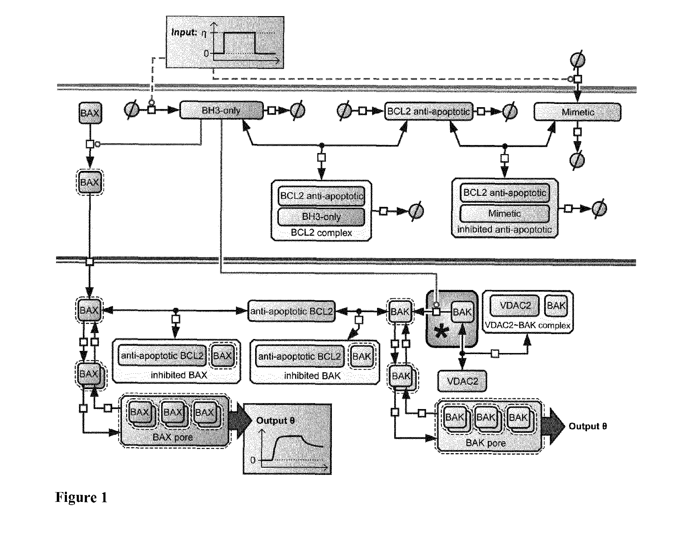

Figure 1 illustrates a model of BCL2 protein interaction during genotoxic stress. The illustration is a Systems Biology Graphical Notation (SBGN) based scheme of the computational model to describe the direct- and indirect activation model from the current literature. In the diagram, the box 'BCL2 anti-apoptotic proteins' represent BCL-2, BCL-(X)

L and MCL-1; the box 'BH-3 only' (red) indicates the genotoxic stress mediators PUMA and NOXA while their hetero-dimers are described by the box 'BCL-2 complex'. The several homo-oligomerisation steps of the effectors BAK and BAX were reduced to one step in the figure. Inactive BAK (purple box, marked with an asterisk *) is only considered in our implementation of the direct activation model. For the indirect activation model the inactive BAK was disregarded (indicated by asterisk) and VDAC2 was assumed to bind directly to the activate form of BAK. Active BAK and BAX or their complexes were framed with an additional dashed border. The box 'Mimetics' (orange) accounts for calculations where drugs such as ABT-737 or Gossypol that mimic the effects of BH3-only proteins were also taken into consideration. As model output, BAK and BAX oligomerisation was assumed to induce pore formation. MOMP was considered to be present upon 10% of the total amount of BAK and BAX being oligomerized to hexamers or higher oligomers (assumed as pores).

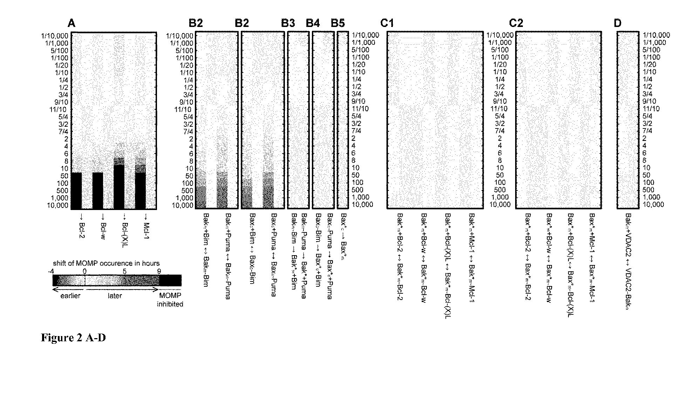

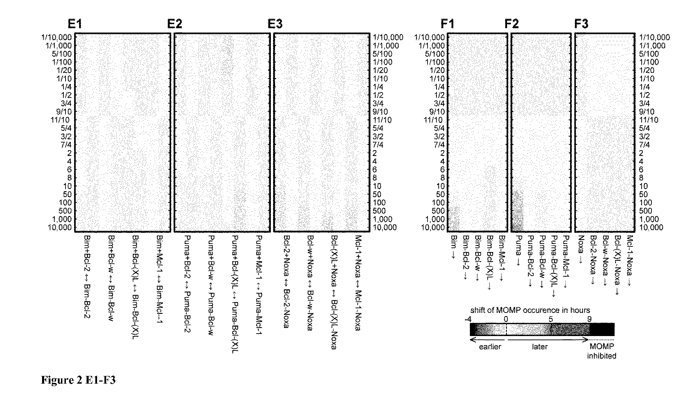

Figure 2 illustrates variations in the time point of MOMP caused by variations in kinetic constants of the model for a typical cell with expression levels of BCL-2, BCL-(X)L, BCL-W and MCL-1 of 100 nM and levels of BAK, BAX and VDAC2 assumed as 500 nM each. **(A)** Changes in the time point of MOMP due to variations of the protein expression (protein turnover) for the anti-apoptotic proteins BCL-2, BCL-(X)L, BCL-W, MCL-1. Variations are equivalent to down or up regulation of these proteins. Of note, MCL-1 and BCL-(X)L down regulation most significantly sensitized these cells to MOMP. **(B1-B5)** Changes in the time point of MOMP caused by changes in the forward and backward constant for BAK and BAX activation are given. A reversible step (B1-B2) for BIM and PUMA binding to BAK and BAX, and irreversible step for BAK and BAX activation are considered (B3-B5). **(C1-C2)** Forward and backward constants for binding of BAK and BAX to anti-apoptotic proteins was changed **(D)** Forward and backward constant for binding of inactive BAK to VDAC2 was changed. **(E)** Forward and backward constant for BIM, PUMA and NOXA to anti-apoptotic proteins. **(F)** Degradation rate of protein and protein complexes was changed.

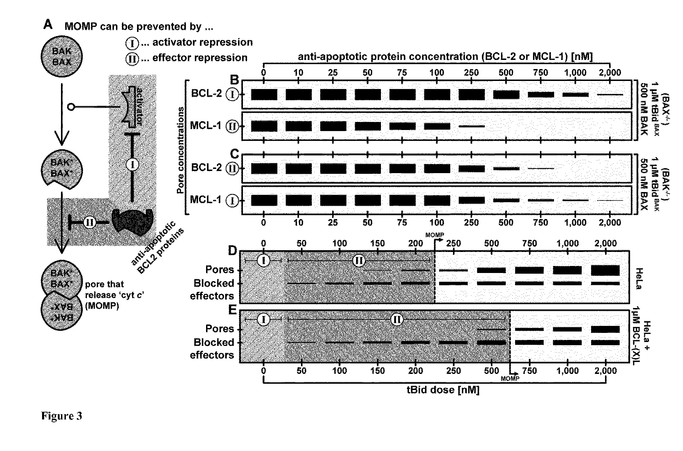

Figure 3 illustrates a model prediction of effector repression being more efficient than repression of activators. **(A)** Sketch of both inhibition types. **(B-E)** Simulated Western Blots where the area of the bar is proportional to maximum amount of pores after t = 96 hours (normalised to an amount of 500 nM of BAX or BAK).

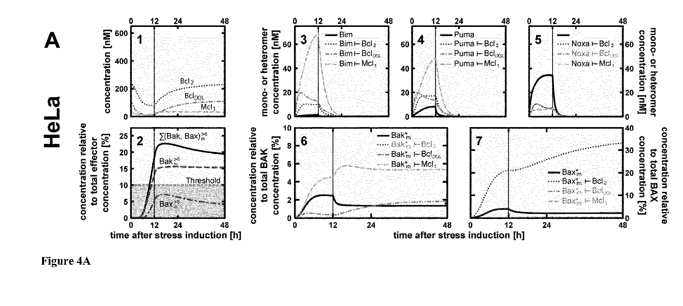

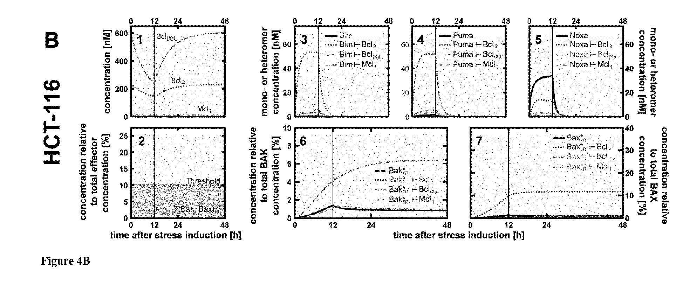

Figure 4 illustrates a direct activation model which gives detailed protein profiles after genotoxic stress induction and cell predictions of MOMP. **(A-B)** Genotoxic stress was modelled to induce the BH-3 only proteins BIM, PUMA and NOXA with a protein dose of 400 nM for 12 hours (vertical dotted lines) for (A) HeLa and (B) HCT-116 wild type cells. Calculated individual profiles of protein complex concentrations are given in each subpanel and denoted by Arabic numbers **(1)** the amount of free anti-apoptotic proteins BCL-2, BCL-(X)L and MCL-1, **(2)** free and active effector monomers of BAK and BAX, **(3)** free BIM and BIM sequestered to anti-apoptotic proteins, **(4-7)** same as (3) for PUMA (4), NOXA (5) active BAK monomers (6), active BAX monomers (7).

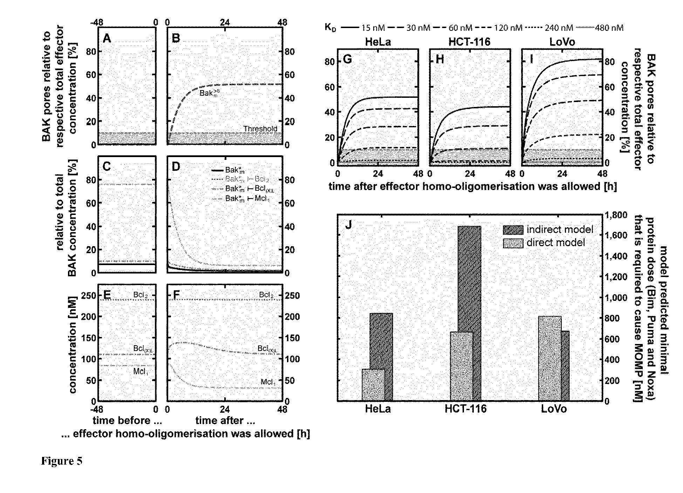

Figure 5 illustrates an indirect activation model which requires a low dissociation of BAK and BAX oligomers and requires a higher stress dose for MOMP induction. **(A)** No pore formation was calculated when oligomerisation was blocked in the model; **(B)** BAK pores induce MOMP once BAK oligomerisation was allowed; **(C)** no change in free and inhibited BAK when oligomerisation was not allowed; **(D)** BAK heterodimers dissociated once BAK oligomerisation was allowed; **(E)** levels of anti-apoptotic proteins and their hetero-oligomers with BAK and BAX were at steady state when oligomerisation was blocked; and **(F)** once BAK oligomerisation was allowed, a different steady state was established reflecting the different amounts of BAK and BAX monomers. **(G-I)** BAK pore formation was calculated for the indirect model under the assumption of higher dissociation of BAK oligomer (an increased backward reaction constant) and for HeLa (G), HCT-116 (H) and LoVo cells. **(J)** Required stress to induce MOMP in HeLa, HCT-116 and LoVo cells in the direct and indirect activation model, the latter assuming a backward constant of 480 nM. The indirect model predicted a higher required stress to induce MOMP in all cells with a maximum level of HCT-116 cells requiring 1.6 $\mu$M of each BIM, PUMA and NOXA.

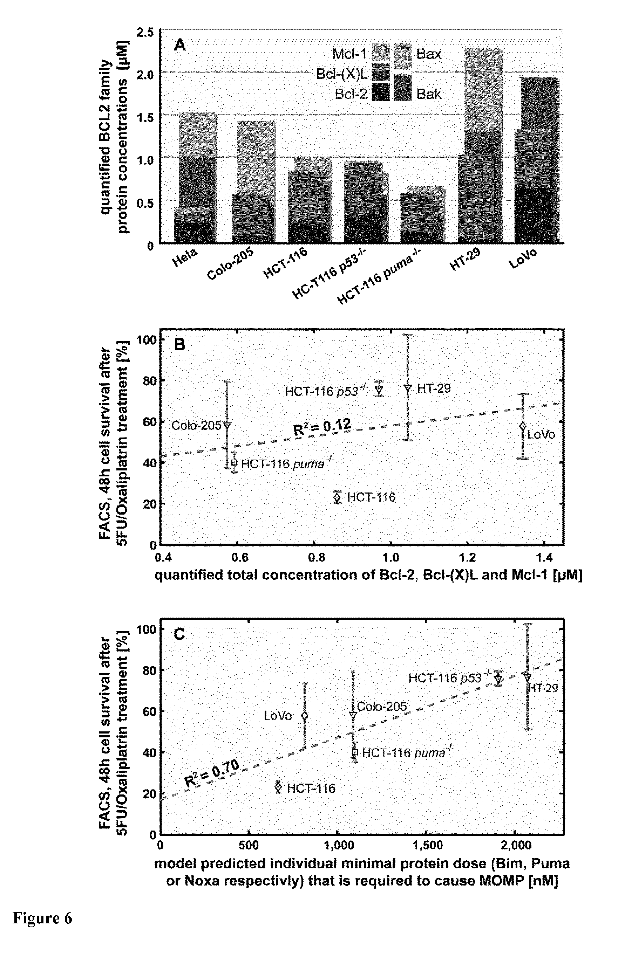

Figure 6 illustrates that a minimal protein dose that is required to induce MOMP is a model predictor of cell sensitivity to undergo apoptosis after genotoxic stress. **(A)** Quantifications of several BCL-2 proteins in the colorectal cancer cell lines Colo-205, DLD-1, HCT-116 (wild *type, p53$^{-/-}$ and puma$^{-/-}$),* HT-29 and LoVo and in HeLa cervical cancer cell lines are given obtained from quantitative Western Blotting as described in the methods section. Besides HCT-116 wild type and mutated cell lines, total expression levels of the apoptotic effectors BAK (dark grey bar with striped texture) and BAX (liked grey bar with striped texture) were higher than the sum of all quantified anti-apoptotic BCL2 proteins BCL-2 (black), BCL-(X)L (dark grey with dotted texture) and MCL-1 (bright grey). **(B)** Cell lines were exposed to 30$\mu$g/ml 5FU and 10$\mu$g/ml Oxaliplatin and the amount of apoptotic cells relative to the total population was detected by FACS. Only a weakly positive trend was observed ($R^2$ = 0.12, Pearson coefficient = 0.35 with significance p = 0.50; Spearman rank coefficient = 0.31, p = 0.56). (C) Fraction of surviving cells from the FACS data in (B) was plotted against the minimal protein dose of BIM, PUMA and NOXA (or only BIM for p53 mutant/deficient cells; only BIM and NOXA for HCT-116 *puma$^{-/-}$* cells) that the model predicted to be necessary for MOMP. Correlation analysis confirmed a significant linear trend (linear regression residual $R^2$ = 0.70, Pearson coefficient = 0.84, with significance p = 0.04).

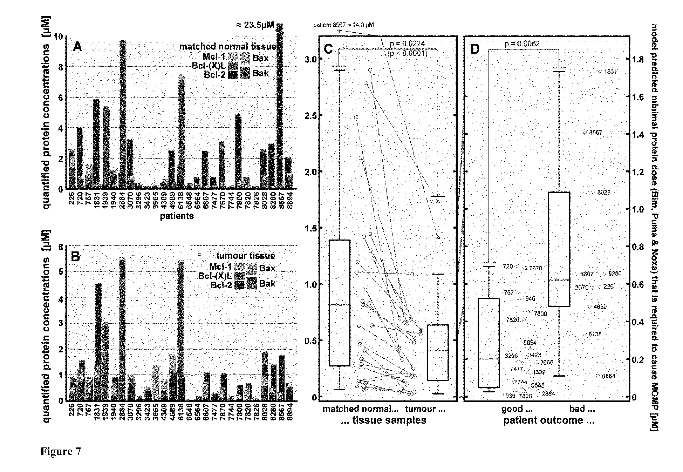

Figure 7 illustrates that a minimal protein dose required for MOMP can be used as predictor of clinical success in CRC patients. **(A-B)** BCL-2 family proteins BAK, BAX, MCL-1, BCL-(X)L and BCL-2 were quantified for tumour (A) and matched normal tissue (B) of 27 Stage II and III colorectal cancer patients by quantitative Western Blotting against HeLa of Figure 6A. Total levels of the pro-apoptotic proteins BAK (dark grey bar with striped texture) and BAX (liked grey bar with striped texture) were higher than the sum of all quantified anti-apoptotic BCL2 proteins BCL-2 (black), BCL-(X)L (dark grey with dotted texture) for most of the patients. No obvious pattern emerged that allowed to assess cell death susceptibility between matched normal (A) and tumour (B) tissue. **(C)** Minimum protein dose of BIM, PUMA and NOXA that is required to induce MOMP was calculated for representative cells of patient matched normal and colorectal cancer (CRC) tumour tissue with absolute protein profiles obtained from (A) and (B). Wilcoxon signed-rank sum test confirmed significance between both groups (p < 0.0001). **(D)** Minimum protein dose of BIM, PUMA and NOXA that is required to induce MOMP may separate patients with favourable and unfavourable clinical outcome. Patients with favourable clinical outcome (left box-plot) required a significantly lower protein dose than patients with unfavourable outcome (left box-plot; Wilcoxon signed-rank sum test p < 0.01). Patient ID numbers according to the Beaumont Hospital patient key are given.

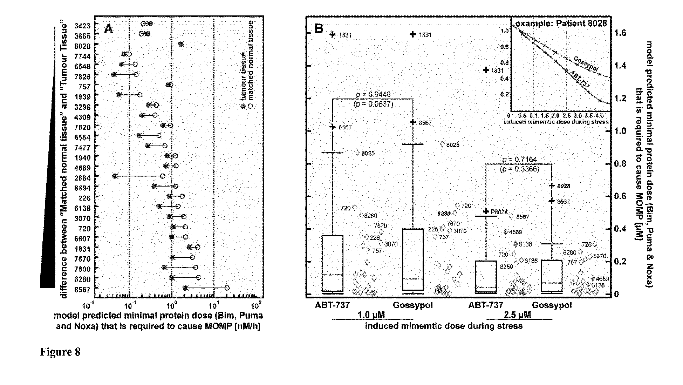

Figure 8 illustrates that systems modelling can act as a clinical tool to determine therapeutic windows and to assess adjuvant treatments. **(A)** Calculated minimal protein dose that is required to induce MOMP may be a potential indicator of patient individual therapeutic windows. Protein dose of BIM, PUMA and NOXA that is expressed upon genotoxic stress is assumed to be a surrogate for the administered chemotherapeutic dose. Differences of the predicted minimum protein dose between matched normal and tumour tissue for each patient as calculated in Figure 7D is indicated by arrows. Arrow lengths indicate differences in protein dose and their direction indicates the presence (arrows to the left) or absence (arrows to the right) of a therapeutic window. (B) Administration of the either of the BH3-only mimetics ABT-737 or Gossypol lower the required dose for chemotherapy. Gossypol or ABT-737 was modelled to be administered over 12hr to a final amount of between 0.5 $\mu$M and 4.5 $\mu$M.

## **Detailed Description of the Drawings**

Materials and Methods

*Model input and readout*

To represent genotoxic stress, the production of the pro-apoptotic BH-3 only proteins BIM, PUMA and NOXA was assumed to initiate a subsequent network of BCL2 protein interactions. The production rates of BIM, PUMA and NOXA were assumed to be identical for all three proteins and modelled by a step function. The height of this function was denoted as protein production rate $\eta$ and varied throughout the study as means to model stress severity. Unless otherwise specified, production of BIM, PUMA and NOXA was modelled to be active for twelve hours, modelled to start at a time point of t = 0hr and end at a time point t = 12hr. To all other time points the production rate $\eta$ was set to zero. The protein production rate $\eta$ multiplied by the duration (12 hours) is defined here as protein dose.

The initial concentrations of the BCL2-family proteins BAK, BAX, BCL-2, BCL-(X)L, MCL-1 were obtained by quantitative Western Blotting in HeLa cells, the colorectal cancer cell lines (COLO-205, DLD-1, HCT-116 (wild type), HCT-116 *puma*[-/-], HCT-116 *p53*[-/-], HT-29 and LoVo), Glioblastoma Multiforme (GBM) cell lines (A172, MZ18, MZ51, MZ256, MZ256, MZ294, MZ304, MZ327, U87, U251, U343 and U373) and all colon cancer patients as described below.

The anti-apoptotic proteins BCL-2, BCL-(X)L, and MCL-1 were modelled to underlie a permanent turnover. To reflect the different concentrations of BCL2 proteins for each cell, cell specific production rates were chosen and together with assumed degradation constants for each protein (Table 2) resulted in equilibrium concentrations of these proteins in the absence of stress. MCL-1 degradation was modelled to drop from 45 minutes to 17 minutes simultaneously with the induction of BIM, PUMA and NOXA production. The model output was the concentration of the BAX and BAK pores. These pores were assumed to be homo-oligomers in complexes equal or larger than hexamers. MOMP was assumed to occur once 10% of total BAK and BAX were homo-oligomerised in pores.

*Mathematically modelling and calculations*

The signalling pathway of the BCL2 family was modelled by creating a set of ordinary differential equation (ODEs) that were based on the law of mass action. Each ODE described the changes in concentration of a single protein or a single protein complex at a time point t. The protein concentrations of modelled proteins at time point t as well as the kinetic constants were used as arguments in each ODE. The ODE of a protein or protein complex was composed from sub-ODEs which were translated from protein reactions **(Table** 1) in which the particular protein (or the complex) was involved as reagent or product. For the translation of the protein reaction to a mathematical equation, a distinction is made at the type of protein reactions between protein degradations, protein turnovers, irreversible reactions as well as reversible reactions.

The degradation rate of a protein or protein complex which was degraded, was calculated by multiplying the protein concentration with the degradation constant $k_{deg}$ **(Table 1A).** The resulting degradation rate was subtracted from the protein concentration of the degraded protein. To determine the protein concentration change caused by a protein turnover, we added the production rate $k_{prod}$ to the degradation rate of the protein that underlay the turnover **(Table** 1B). The equilibrium concentration of the protein which underlay a protein turnover was calculated by dividing its production rate $k_{prod}$ by its degradation constant $k_{deg}$ **(Table 1B).** The change in concentration due to an irreversible reaction was modelled by multiplying the reagent protein concentration by the reaction constant k. The concentration of the involved reagent is reduced by the protein change, whereas the resulting product was increased by the protein change **(Table 1C).** In the case of more than on reagent, the product of all reagent concentration was multiplied with the reaction constant k **(Table 1D).** Reversible reactions were modelled by considering a forward and backward reaction. The protein changed caused by the protein forward reaction was modelled to be the product of involved reagents multiplied with the forward constant $k_{forward}$. This change was added to the concentration of the product and subtracted from the concentrations of each reagent. The change caused by the backward reaction was modelled to be the concentration of the product protein complex (or protein) multiplied by the backward constant $k_{backward}$. The resulting protein concentration change was added to each reagent concentration and subtracted from the product concentration **(Table 1E).**

*Modelled BCL2 protein interactions*

In the model of the present invention, VDAC2, BAK and BAX monomers, BAK and BAX hetero-dimers, and BAK and BAX homo-oligomers were assumed to be stable and not to get degraded. All other proteins were subjected to degradation **(Table** 2). The anti-apoptotic proteins BCL-2, BCL-(X)L, BCL-W and MCL-1 were the only protein that underlay a constant protein production. For each cell, the production rates are set to values that hold the cell specific initial concentrations (Figure 6A) of the anti-apoptotic BCL2 proteins in the absence of stress. The cell, as well as BCL2 protein specific production rate, is determined by dividing the cell specific initial concentration by the anti-apoptotic BCL2 protein specific degradation constant $k_{deg}$.

The core of the model was the specific binding of anti-apoptotic proteins BCL-2, BCL-(X)L and MCL-1 to the pro-apoptotic proteins BAK, BAX, BIM, tBID, PUMA and NOXA (see **Table 3).** The binding affinities are described by the dissociation constant $K_D$. A low $K_D$ value indicates high protein affinity whereas a high $K_D$ value indicates low protein affinity. The dissociation constants were frequently used in literature whereas we did not obtain all required forward and backward kinetic constant. However, the paper by Chen *et. al.* (L. Chen, S. N. Willis, A. Wei, B. J. Smith, J. I. Fletcher, M. G. Hinds, P. M. Colman, C. L. Day, J. M. Adams, and D. C. S. Huang, 'Differential targeting of prosurvival Bcl-2 proteins by their BH3-only ligands allows complementary apoptotic function', Mol. Cell, vol. 17, no. 3, pp. 393-403, Feb. 2005) determined dissociation constants $K_D$ as well as the forward constants $k_{forward}$ and the backward constants $k_{backward}$ for the binding of the anti-apoptotic proteins BCL-2, BCL-(X)L, BCL-W and MCL-1 to BIM and were assumed for this model. The backward constant of the reversible binding of the anti-apoptotic BCL-2, BCL-(X)L, BCL-W and MCL-1 to BAK, BAX, PUMA, tBID and NOXA is assumed in this model to be identical to the backward constant of binding of BCL-2, BCL-(X)L, BCL-W and MCL-1 to BIM, in respect of BCL-2, BCL-(X)L, BCL-W and MCL-1. The associated forward constants $k_{forward}$ were determined by dividing the assumed backward constants by the obtained dissociation constants.

Inactive $BAX_c$ was modelled to be initially present only in the cytosol and to translocate into the mitochondrial outer membrane (MOM) after its activation **(Table 4A-C).** It is assumed that no interaction of inactive $BAX_c$ with anti-apoptotic BCL2 family proteins. BH3-only proteins BIM, PUMA and tBID were able to bind to inactive $BAX_c$. A dissociation constant $K_D$ of 100 nM, a backward constant of 2.57E-04 s$^{-1}$ (which correspond with a half-life time of 45 minutes) and a forward constant of 2.57E-06 nM$^{-1}$ s$^{-1}$ are assumed. The constant of the enzymatic activation of inactive $BAX_c$ in the hetero-dimer was assumed to be 1.16E-01 s$^{-1}$ with a subsequent instant dissociation from the dimer. The dissociated $BAX_c$* is modelled to translocate to the MOM with a constant of 1.16E-01 s$^{-1}$ (which is equivalent to a half-life time of six seconds).

Since BAK and BAX are similar in form and function, the activation of BAK is modelled in the direct activation model with the same reactions and the same kinetic constants (see **Table 4A-B).** Based on the fact that BAK is exclusively located to the MOM, BAK translocation from the cytosol to the membrane was not modelled. In contrast to BAX, BAK is modelled to interact with the Voltage-dependent anion-selective channel protein 2 (VDAC2). In each calculation, VDAC2 was assumed to be expressed with the same concentration as BAK in the cell. In the direct activation model, only inactive BAK is assumed to bind to VDAC2 with a dissociation constant of 1,000 nM. The VDAC2~BAK dimer was assumed to dissociate with a half-life time of five minutes (backward constant $k_{backward}$ = 2.31E-03 s$^{-1}$). The forward constant $k_{forward}$ was set to 2.31E-06 nM$^{-1}$ s$^{-1}$ (see **Table 4D).** In the indirect activation model, the BAK activation step was removed and only constitutive active BAK* was assumed. For the indirect activation model we assumed that VDAC2 binds to active BAK* with the same constants as in the direct activation model. It was assumed that the cytosolic BAX activation step was still required in the indirect activation model.

In both models (direct as well indirect activation model) active BAK* and BAX* was modelled to homo-oligomerise to dimers. The dimers further homo-oligomerised to larger homo-oligomers (see **Table 5).** For the effector binding, a $K_D$ of 15 nM and a decay time of the pores of 1hr ($k_{backward}$ = 1.93E-04 s$^{-1}$ → $k_{forward}$ = 1.28E-05 nM$^{-1}$ s$^{-1}$) was assumed. BAK and BAX homo-oligomerisation was modelled up to twelve proteins in one homo-oligomer. In this model, hexamers or larger homo-oligomers were assumed to be pores that cause the mitochondrial outer membrane permeabilisation (MOMP) once 10% of total effectors homo-oligomerised to pores.

Proteins of the BCL2 protein family were assumed to be mainly located and to interact at the MOM. As the only exception, $BAX_c$ was assumed to have a cytosolic fraction which was modelled to be inactive and only able to interact with the BH3-only proteins, but not with anti-apoptotic proteins.

To study the effect of BH3 mimetics, further ODEs that characterised ABT-737 and Gossypol binding (see **Table 6)** were included. Each mimetic was assumed to bind to the anti-apoptotic proteins BCL-2, BCL-(X)L and MCL-1. The required mimetic specific dissociation constants $K_D$ were taken from literature (G. Lessene, P. E. Czabotar, and P. M. Colman, 'BCL-2 family antagonists for cancer therapy', Nat Rev Drug Discov, vol. 7, no. 12, pp. 989-1000, Dec. 2008). Backward constants $k_{off}$ of 1.93E-04 s$^{-1}$, which are equivalent to a decay half-life time of one hour for a dimer, were assumed. Degradation of the mimetics and the hetero-dimers were also modelled. A degradation half-life time of two hours for ABT-737 and four hours for Gossypol were assumed. For the hetero-dimers, it was assumed to be in a similar range as the other hetero-dimers (see **Table 6B).**

For each cell, a steady state to determine the BCL2 family concentrations in the absence of stress was calculated. The resulting steady state was assumed as initial state for the subsequent calculation in the presence of stress. To determine the steady state in the indirect activation model, the BAK and BAX homo-oligomerisation in the steady state calculation was turned off. Effector homo-oligomersiation was reactivated in the subsequent calculation.

For implementation and solving of the ODEs, MATLAB 7.3 (MathWorks, USA, R2007b, 7.5.0.342) and its function *ode15s* was used.

*Qualitative analysis of activator and effector inhibition*

Two recently developed tBID chimeras, tBID$^{BAK}$ and tBID$^{BAX}$ whose tBID BH3 domains were replaced with that of BAK or BAX were modelled. The specific binding characteristic of these chimeras allowed studying how easy anti-apoptotic proteins that either bind to activator or effector (BAK and BAX) can be de-repressed from their binding partners. The binding affinities (forward and backward constants) of tBID chimeras to the anti-apoptotic protein were assumed to be the same as for BAK or BAX. For the activation of BAK and BAX by tBID chimeras, the same kinetics as for wild type tBID activating of the effectors were assumed. For the degradation of the chimera monomers and of the heterodimers between chimera and anti-apoptotic proteins, a half-life time of 75 minutes was assumed, identical to the one considered for tBID.

To study the inhibition of either the effector or activator, cells with either 500 nM of BAK or BAX were assumed. For BAK and BAX expressing cells, two sets of calculations each were performed. In each set, the concentration of a single anti-apoptotic protein, BCL-2 or MCL-1, was set to 0; 10; 25; 50; 75; 100; 250; 500; 750; 1,000 and 2,000 nM and performed a calculation to determine the maximum amount of BAK or BAX pores within 48 hours. In the BAK expressing cells, a protein dose of 1 $\mu$M tBID$^{BAX}$ chimeras was applied in each calculation, and in the BAX expressing cells a protein dose of 1 $\mu$M tBID$^{BAK}$ chimeras was applied in each calculation.

To inhibit only the effector, we varied MCL-1 in BAK expressing cells and BCL-2 in BAX expressing cell. This was consistent with the assumption that each anti-apoptotic protein only bound to the effector (BAK or BAX) and not to the activator (tBID$^{BAX}$ or tBID$^{BAK}$). To inhibit only the activator, MCL-1 in BAX expressing cells were varied and BCL-2 in BAK expressing cells were varied.

Inhibiting of only the activator or the effector is consistent with the two modes, Mode 1 (activator inhibition) and Mode 2 (effector inhibition) of Llambi *et. al.* (F. Llambi, T. Moldoveanu, S. W. G. Tait, L. Bouchier-Hayes, J. Temirov, L. L. McCormick, C. P. Dillon, and D. R. Green, 'A unified model of mammalian BCL-2 protein family interactions at the mitochondria', Mol. Cell, vol. 44, no. 4, pp. 517-531, Nov. 2011).

For the second model calculation wild type Hela and BCL-(X)L over expressing HeLa cells (additional 1 $\mu$M) were assumed to be exposed to a protein dose of tBID to either 0, 50, 100, 150, 200, 250, 500, 750, 1,000 and 2,000 nM, over 12 hours. The maximal amount of pores and the maximal amount of effectors bound to anti-apoptotic BCL2 proteins (inhibited effectors) were determined.

Results for both calculations were plotted as simulated Western Blot. The area of the bar was proportional to the amount of pores and normalised to 500 nM in the first calculations (see Figure 3B) and 1,500 nM in the second calculations (see Figure 3C).

*Cell culture*

Cell culture for COLO-205, DLD-1, HCT-116 (wild type), HCT-116 *puma$^{-/-}$,* HCT-116 *p53$^{-/-}$,* HT-29 and LoVo was prepared as previously specified in: Concannon CG, Koehler BF, Reimertz C, Murphy BM, Bonner C, Thurow N, Ward MW, Villunger A, Strasser A, Kögel D, Prehn JH. Apoptosis induced by proteasome inhibition in cancer cells: predominant role of the p53/PUMA pathway. Oncogene. 2007 Mar 15;26(12):1681-92.

*Patient cohort*

Colorectal cancer patient tissue was collected and stored from the Departments of Surgery, Gastroenterology and Pathology, Beaumont Hospital, Dublin, Ireland. Matched adjacent normal colorectal tissue was available for all of these patients. Clinical follow-up was obtained through a review of medical records by a dedicated clinical research nurse. For classification purposes, patients without disease recurrence and/or cancer mortality within 4 years were classified as a *good outcome;* patients who recurred/died from colorectal cancer were classified as *bad outcome.* Patients with hereditary forms of colorectal cancer were excluded. Ethical approval has been obtained for this study by the Beaumont Hospital Ethics (Medical Research) Committee and informed consent was obtained from all patients. Tissue was stored for use as snap frozen (-80°C) or in RNAlater (Ambion, Abington, UK) (-20°C).

*Absolute Protein Quantifications*

Patient tissue was lysed in 500μL ice cold tissue lysis buffer (50mmol/L HEPES (pH 7.5), 150mmol/L NaCl, 5mmol/L Na-EDTA) and protease inhibitor (Calbiochem, Hampshire, UK) followed by mechanical homogenization on ice. Samples were centrifuged at 14,000 x g for 10 minutes, supernatant collected and stored at -80°C. HeLa cell lysates were prepared in an identical manner. Protein concentrations were determined using micro BCA assay (Pierce, Rockford, IL). Western blotting of total protein (20 μg) was carried out as described previously (M. Rehm, H. J. Huber, H. Dussmann, and J. H. M. Prehn, 'Systems analysis of effector caspase activation and its control by X-linked inhibitor of apoptosis protein', EMBO J., vol. 25, no. 18, pp. 4338-4349, Sep. 2006). Images were acquired at 16 bit dynamic range using a LAS-3000 Imager (FUJIFILM UK Ltd.Systems, Bedford UK). Densitometry was performed using ImageJ software. As there was demonstrable difference in expression of β-actin  expression between tumour and matched normal tissue, β-actin was used for normalization of intensity for each protein. β-actin normalized intensities were used to determine tumor to matched normal tissue ratios in protein expression. For quantitative Western blotting, standard curves from recombinant human proteins and HeLa cell extracts were run concurrently with tumor samples to ensure linearity of the signal detection range. Protein concentrations in patient samples were determined by comparison to signals from HeLa cell extracts. Primary antibodies to MCL-1 (1:250; BD Biosciences), BCL-2 (1:100; Santa Cruz Biotechnology, Inc.), BCL-(X)L (1:250; Santa Cruz Biotechnology, Inc.) and β-actin (1:20,000; Sigma Aldrich, Dublin, Ireland) were mouse monoclonal. Antibodies to BAK (1:100; Santa Cruz Biotechnology, Inc.) and BAX (1:100; Upstate Biotechnology) were rabbit polyclonal. Images were contrast adjusted and converted to 8-bit dynamic range for presentation purposes.

*Determining of the minimal protein dose that is required for MOMP*

The minimal protein dose $\eta_{min}$ (BIM/PUMA/NOXA) that is sufficient to cause MOMP was determined by an iterative approximation algorithm for each cell. MOMP was assumed once 10% of total effectors were bound to pores. An initial protein dose $\eta_0$ of 1 μM/s was assumed. The initial step size for the iteration $\Delta\eta_0$ was set to the half value of the initial stress $\eta_0$. It was then determined whether or not the model predicted MOMP in that particular cell, under the initially assumed stress. If MOMP was not predicted, the assumed protein dose was increased by the step size $\Delta\eta_0$ ($\eta_{i+1}$ = $\eta_i$ + $\Delta\eta_0$). When the protein dose was sufficient to cause MOMP, the assumed protein dose was decreased by the step size $\Delta\eta_0$ ($\eta_{i+1}$ = $\eta_i$ - $\Delta\eta_0$). Subsequently, a new step size was set to the half of the current value of $\Delta\eta_{i+1}$ = $\Delta\eta_i$ / 2. Calculations were repeated until the step size was smaller than $10^{-3}$ nM/s. The last protein dose $\eta_i$ was assumed to be the smallest required protein dose that is sufficient to cause MOMP.

*Sensitivity analysis*

All model constants (kinetics) were changed by a factors of the set [$10^{-4}$; $10^{-3}$; $5*10^{-3}$; $10^{-2}$; $5*10^{-2}$; $10^{-1}$; 1/4; 1/2; 3/4; 9/10; 11/10; 5/4; 3/2; 7/4; 2; 4; 6; 8; $10^1$; $5*10^1$; $10^2$ $5*10^2$; $10^3$; $10^4$] and the resulting changes in the time point of MOMP were investigated. For all calculations a protein dose (BIM/PUMA/NOXA) of 1 μM was modelled to be present for 12 hours. Calculations were made for a typical cell line that expressed 100 nM of BCL-2, of BCL-(X)L, BCL-W and MCL-1; and 500 nM of BAK, BAX and VDAC2. The first MOMP occurrence within 96 hours was taken as result. In advance of each calculation that determine MOMP occurrence, the new steady state in absence was determined after the kinetic was changed.

*Statistical analysis of results*

A correlation analysis was performed to determine the relationship between model prediction and amount of cell survival in a cell population of a CRC or GBM cell line subjected to stress (5FU/Oxaliplatin, survival obtained by FACS). The minimal protein dose that the model predicted that is required to induce MOMP in a prototypical cell of above cell line was correlated with the amount of cell survival subsequent to drug administration. As comparison, the amount of total concentration of all anti-apoptotic proteins was correlated to the same amount of cell survival. For all above studies, the grade of linear dependency of two variables was assessed by the Pearson's correlation coefficient. For analysis the MATLAB 7.3 (MathWorks, USA, R2007b, 7.5.0.342) function *corr* was used. A good correlation as predicted by the methods required the correlation coefficient being close to or equal one.

For assessing whether the model predictor (minimal protein dose that was required to induce MOMP) was able to separate between good and bad outcome for CRC patients the Wilcoxon rank-sum test was applied using the MATLAB routine *ranksum.* For comparing whether the same model predictor indicates that the protein dose required to induce apoptosis in tumour tissue and in matched normal tissue are independent from each other, the Wilcoxon signed rank-sum test for paired data sets was applied and the MATLAB routine *signrank* was used therefore.

**Results**

*An* in silico *model for studying MOMP in cancer cell lines in response to genotoxic stress*

A computational model was constructed that allows a study of temporal BCL2 protein profiles and an investigation of how pro- and anti-apoptotic BCL2 interact to induce MOMP in response to genotoxic stress (Figure 1) in individual cell lines. Genotoxic stress was assumed to induce the BH3-only proteins BIM, PUMA and NOXA (Figure 1. Red boxes), leading to an interaction network of pro- and anti-apoptotic proteins, resulting of BAK and BAX homo-oligomerisation (blue boxes) and finally MOMP (grey box, denoted as output). Oligomers larger or equal than hexamers were defined as pores which caused MOMP once 10% of BAK or BAX were bound in oligomers. Through engagement of BH3-only proteins and of BAK and BAX, the anti-apoptotic BCL2 proteins BCL-2, BCL-(X)L and MCL-1 (green boxes) were assumed to prevent MOMP. Each of these anti-apoptotic proteins was modelled to be produced at a constant rate and to bind to their respective pro-apoptotic partners with a specific affinity (L. Chen, S. N. Willis, A. Wei, B. J. Smith, J. I. Fletcher, M. G. Hinds, P. M. Colman, C. L. Day, J. M. Adams, and D. C. S. Huang, 'Differential targeting of prosurvival Bcl-2 proteins by their BH3-only ligands allows complementary apoptotic function', Mol. Cell, vol. 17, no. 3, pp. 393-403, Feb. 2005) (Table 3). Hetero-dimers consisting of pro-apoptotic and pro-survival proteins were subjected to degradation (Table 3). MCL-1 expression was assumed to be decreased upon onset of stress due to enforced ubiquitination (Table 2; for changes of MCL-1 half life time, see Table 2 and methods).

To contribute to the ongoing controversy, that is whether an additional activation step of BAK and BAX by some BH3-only proteins ('activators', here only PUMA, BIM and in some cases tBID) is necessary for BAK and BAX activation or not, we implemented this step as an option in our model (solid red line). Whether or not this option was implemented led to two model variants which are consistent with the 'direct activation model' (A. Letai, M. C. Bassik, L. D. Walensky, M. D. Sorcinelli, S. Weiler, and S. J. Korsmeyer, 'Distinct BH3 domains either sensitize or activate mitochondrial apoptosis, serving as prototype cancer therapeutics', Cancer Cell, vol. 2, no. 3, pp. 183-192, Sep. 2002) and the 'indirect activation model' (D. C. Huang and A. Strasser, 'BH3-Only proteins-essential initiators of apoptotic cell death', Cell, vol. 103, no. 6, pp. 839-842, Dec. 2000) from literature. In both variants, BAX is assumed to translocate to the mitochondria upon activation, while BAK is assumed to be constitutively present at the mitochondrial membrane where inactive BAK (or constitutively active BAK in the indirect activation model) was modelled to bind additionally to the inhibitor VDAC2 (Table 4).

To make the model of the present invention applicable to the study of novel chemotherapeutic drugs that mimic pro-apoptotic BCL2 proteins the molecular interactions of the BH3-only mimetics ABT-737 and Gossypol were integrated. Consistent with their biochemical function, mimetics were modelled to sequestered anti-apoptotic BCL2 proteins (Table 6).

*Sensitivity analysis suggests inhibitory role of MCL-1 and BCL-XL as larger than BCL-2 and BCL-W*

While basing the kinetic model parameter for protein-interactions on experimental data from the current literature, such kinetic data was often taken from studies within an aqueous instead of the lipid environment of the mitochondrial membrane. To this end, a medium level genotoxic stress was applied that leads to a BIM, PUMA and NOXA production of 1 $\mu$M total production of each of BIM, PUMA and NOXA over 12 hr (defined as protein dose) to a typical cell. This typical cell was used to express 100 nM each of BCL-2, BCL-W, BCL-(X)L and MCL-1 and 500 nM of each BAK, BAX and VDAC2. MOMP in this cell was observed 6 hr after stress induction.

First, it was investigated how a change of protein turnover of the anti-apoptotic proteins BCL-2, BCL-W, BCL-

(X)L and MCL-1 changed the time point of MOMP. For each protein, the protein production rate (technically the forward kinetic constant) and the protein degradation constant (backward kinetic constant) was changed by the respective factor from the interval [0.0001, 1,000]. As expected, increasing the rate of protein production (equivalent in increasing the steady state concentration of the investigated protein) led to a later time point of MOMP until MOMP was inhibited (Figure 2A, black bars at bottom in the first lane of each protein). Decreasing protein production and, therefore, protein concentration led to a slight increase in time before MOMP occurred (1hr, Figure 2A, yellow bars in the first lane of each protein heading to top) with the earliest occurrence when BCL-(X)L levels were lowest. In turn, increasing or decreasing the degradation constant of each of these proteins by the same factors (Figure 2A, second lane for each protein, heading top for increase and bottom for decrease) led to a later or earlier occurrence of MOMP, respectively. Together, results from Figure 2A suggest that changes in BCL-(X)L and MCL-1 levels had a more decisive effect on time point of MOMP.

As this activation was modelled in a two-step procedure, in which BIM and PUMA bound to inactive BAK and BAX, and eventually released them as active proteins, changes of the parameters that describe both steps (Figure 2B1-B2 for step 1 and 2B3-B5 for step 2) were investigated. Strikingly, increased BIM and PUMA binding to BAK and BAX (increased forward constant) rigorously accelerated MOMP by 1hr to more than 3hr (Figure 2B1-B2, heading to bottom). Conversely, decreased BIM and PUMA binding to BAK and BAX only modestly delayed MOMP (Figure 5B1-B2, heading to top) suggesting that the assumed reference binding affinity (the experimentally determined association constant of Chen *et al.*) was high enough to guarantee robust BAK and BAX activation. Strikingly, increasing or decreasing kinetic constants of the second step did not severely alter the time of MOMP suggesting the first activation step to be regulating and suggesting that activation speed is not the bottleneck / critical path in the network.

Once activated, active BAK and BAX may be bound by anti-apoptotic proteins which prevent BAK and BAX oligomerisation. Changing the forward and backward constant of these complexes with BAK (Figure 2C1) and BAX (Figure 2C2), however, also did not lead to a severe change in the kinetics of MOMP (indicated by large white areas), corroborating the finding that the first activation step is the regulating one as mentioned above.

The anti-apoptotic proteins BCL-2, BCL-(X)L and MCL-1 may bind BIM, PUMA, and NOXA in a reversible manner and we therefore investigated how changes of the forward and backward constant of this binding influenced the time point of MOMP. However, changing BIM and PUMA inhibition by their anti-apoptotic binding partners did also not markedly change time of MOMP occurrence suggesting the reference binding affinity as low (Figure 2E1-E3).

Finally, it was analysed how a change in protein degradation influenced the time point of MOMP. Indeed, it is known that protein life time is a significant variable in oncogenesis and cancer treatment with several drugs that target proteasomal degradation currently being in clinical trial. The decreased half-life time of PUMA and BIM delayed the time point of MOMP by up to 4hr. In contrast, changes in life time of hetero-oligomers of BIM and PUMA did not markedly affect time of occurrence of MOMP. Finally, degradation of free BIM and PUMA, but not the degradation of their hetero-dimers, was found to exercise influence with increased BIM and PUMA degradation delaying the time point of MOMP by up to 4hr.

In conclusion, the results suggest that the amount of anti-apoptotic proteins that get produced as well as the binding affinity of the activators BIM and PUMA to BAK and BAX are the most decisive regulator of apoptosis and that MCL-1 and BCL-(X)L are slightly more important regulators in this scenario.

*Repression of apoptotic effectors is more effective to prevent MOMP than repression of activators*

Anti-apoptotic BCL2 proteins are promiscuous inhibitors of apoptosis as they bind to apoptotic effectors as well as to BH3-only activators and sensitizers. Using chimera proteins of the activator tBID that mimicked the BH3-domain of either BAK and BAX (tBID[BAK] and tBID[BAX]), Llambi *et. al.* (F. Llambi, T. Moldoveanu, S. W. G. Tait, L. Bouchier-Hayes, J. Temirov, L. L. McCormick, C. P. Dillon, and D. R. Green, 'A unified model of mammalian BCL-2 protein family interactions at the mitochondria', Mol. Cell, vol. 44, no. 4, pp. 517-531, Nov. 2011) recently proposed that stress induction and expression of BH3-only proteins overcomes this apoptosis repression in a two-step procedure. They exploited the fact that tBID[BAK] and tBID[BAX] selectively bound to a different set of anti-apoptotic proteins (MCL-1 for tBID[BAK], BCL-2 for tBID[BAX]; denoted as activator repression). Their experiments provided evidence that at first BH3-only activator proteins are de-repressed from their binding to anti-apoptotic proteins (activator repression; indicated with (I) in Figure 3A) in order to activate apoptosis effectors BAK and BAX. Since active BAK and BAX are inhibited by anti-apoptotic proteins (effector repression; indicated with (II) in Figure 3A), they confirmed that a second de-repression step is necessary to liberate active BAK and BAX to enable their homo-oligomerisation and for MOMP inductions. They further proposed that effector repression is more efficient to inhibit MOMP than activator repression (i.e. can be more readily repressed), and therefore, both de-repression steps are performed sequentially.

In order to validate the model of the present invention qualitatively, the results from the model of the present invention were compared against the experimental results of *Llambi et al.* To model their experimental systems of mouse liver cells with either BAK or BAX present, it was assumed that cells with 500 nM of the respective effector being present and the other one being absent. BAK deficient BAK[-/-] cells were modelled to be exposed to the chimera tBID[BAK] and BAX$^{-/-}$ cells exposed to tBID$^{BAX}$. It was further modelled that cells take up its respective tBID chimera of 1 $\mu$M from the extracellular medium over 12 hr. The unique binding properties of tBID$^{BAK}$ and tBID$^{BAX}$ allowed selectively studying activator and effector repression by using specific anti-apoptotic proteins. In particular, similar to Llambi et al., a case where tBID chimera only inhibited the respective activator and not the effector (denoted as (I)) was studied. This was done by modelling BAX$^{-/-}$ cells (expressing BAK) to be exposed only to tBID$^{BAK}$ and MCL-1 and by modelling BAK$^{-/-}$ cells (expressing BAX) be exposed only to tBID$^{BAX}$ and BCL-2. In turn, effector repression was investigated by assuming either BAX deficient cells to be exposed to tBID$^{BAK}$ and MCL-1 or BAK deficient cells to be exposed to tBID$^{BAX}$ and BCL-2. For all simulations BCL-X(L) and BCL-W were disregarded and set to zero.

With these specific settings, the amount of BAK (or BAX) pores in BAX$^{-/-}$ (or BAK$^{-/-}$) cells under presence of either only BCL-2 or only MCL-1 and under different initial concentrations ranging from 0 to 2 $\mu$M were calculated. Results for BAX$^{-/-}$ (Figure 3B) and BAK$^{-/-}$ (Figure 3C) cells are depicted by an in silico mock-up Western Blot where the area of the bar is proportional to the calculated maximum amount of pores that is present during a period of 96 hr. As can be seen, in both cells less anti-apoptotic protein is required to prevent pore formation when they bind to the effector (II) than when they bind to the activator (I). These results suggest that activator repression by pro-apoptotic proteins is less efficient than effector repression and are consistent with the results presented in Llambi et al.

An investigation of how realistic cells overcome both types of repression ((I) and (II)) upon stress induction was performed. HeLa cells and BCL-(X)L over-expressing HeLa cells as studied in Llambi et al. were considered and assumed that tBID is expressed with different doses that indicate different amount of stress (e.g. different doses of UV exposure). Indeed, from Figure 3D (HeLa) and 3E (HeLa over-expressing cells) it was noted that with tBID doses lower than 50 nM (both cells), no hetero-dimers of anti-apoptotic proteins with active BAK and BAX (blocked effectors) were observed (activator inhibition, light grey shaded area). After tBID dose was increased, first calculations showed active BAK and BAX to be bound to anti-apoptotic proteins (blocked effectors). This inhibition prevented pore formation and kept the amount of pores lower than the MOMP threshold (dark grey region 'II'). In conclusion, the model of the present invention successfully resembled experimental findings that activator binding by anti-apoptotic proteins is less effective than effector binding and that both modes are activated sequentially in single cells.

*The direct activation model predicts HeLa cells as susceptible and HCT-116 cells as resistant to MOMP under milder genotoxic stress*

The results of the individual protein profiles over time for HeLa cells are given in Figure 4A. As can be seen in subpanel Figure 4A3 and 4A4, only a small fraction of the totally produced BIM and PUMA (solid line in both graphs) remains free while other BIM and PUMA fractions were bound to anti-apoptotic proteins (dotted, dashed-dotted and dashed for BIM and PUMA binding to BCL-2, BCL-X(L) and MCL-1, respectively). In addition, both free and inhibited PUMA were subjected to rapid degradation such that only a fraction of the 400 nM becomes detectable (95 nM in total for BIM and 90 nM for PUMA). Unlike BIM and PUMA, a more significant fraction of NOXA was free (solid line, Figure 4A5). Free BIM and PUMA led to an activation of BAK and BAX. Activated BAK and BAX were mostly inhibited by anti-apoptotic proteins. According to their preferential binding, MCL-1 and to a lesser extent BCL-X(L), inhibited BAK (Figure 4A5, dashed and dashed-dotted line) while BCL-2 predominantly bound to activated BAX (Figure 4A7, dotted line). Nevertheless, a significant amount of free BAK and BAX remained free (solid lines Figure 4A6 and Figure 4A7, respectively).

As a result of their binding to BIM, PUMA and NOXA as well as to BAK and BAX, BCL-2 levels depleted from 240 nM to 80 nM, BCL-X(L) and from 110 nM to 10 nM. Likewise, due to enforced degradation and binding to BIM, PUMA and NOXA, MCL-1 levels from 80 nM to 30 nM at time point of 12 hr (Figure 4A1 dotted, dashed-dotted and dashed line, respectively). Finally, free BAK and BAX eventually oligomerised such that the total amount of BAK and BAX pores exceeded the 10% threshold after 8hr and 36min of total amounts of BAK and BAX (Figure 4A2, solid line) which was assumed as MOMP criterion. Importantly, pore formation was driven by BAK oligomerisation (dashed line) while BAX oligomers alone would not suffice to induce MOMP (dashed-dotted), suggesting that BAK$^{-/-}$- HeLa cells may be stable against MOMP under the particular assumptions of MOMP threshold of 10%.

As HCT-116 cells showed more pronounced expressions of the anti-apoptotic protein BCL2 and a lower expression in BAK and BAX (see Figure 2), it was investigated how these cells would react to the same dose of stress of 400 nM each BIM/PUMA/NOXA (Fig. 4B). Similar to HeLa cells, only little B-IM and PUMA was free (Figure 4B3 and Figure 4B4, solid lines). While BIM mainly bound to BCL-2 (dotted line in Figure 4B3), PUMA mainly bound to BCL-X (L) (dashed-dotted line in Figure 4B3). Similar to HeLa cells, significant amounts of free NOXA were available (Figure 3B6, solid line for free NOXA, dotted, dashed and dashed-dotted for NOXA bound to MCL-1, BCL-2 and BCL-X(L), respectively). Due to the high amount of anti-apoptotic proteins, only little active BAK and BAX (less than in HeLa cells) remained free (Figure 4B6 and Figure 4B7, solid lines). The binding to pro-apoptotic proteins led to a depletion of the anti-apoptotic proteins BCL-X(L), BCL-2 and MCL-1 at t = 12 h (Figure 4B1, dashed-dotted, dotted and dashed lines). As a consequence of the small amount free and active BAK and BAX, neither BAK nor BAX pores were present (Figure 4B4) even after 48hr. In conclusion, using cell line specific expression levels of BCL2 family proteins the model of the present invention can predict cell line specific susceptibility to MOMP.

*Validity of the indirect activation model requires a low dissociation of effector oligomers and therefore requires a higher stress dose for MOMP induction*

In contrast to the direct activation model, the indirect model does not assume an activation step for BAK. In addition, in several cell lines the amount of pro-apoptotic BCL2 family members can be severely higher than the amount of anti-apoptotic proteins (Figure 6A). In HeLa cells in the absence of stress, it was found that BAK pores were obtained once BAK oligomerisation was allowed in the model (Figure 5AB, dashed line for BAK pores). This result suggests that repression of BAK by binding to anti-apoptotic proteins was not sufficient to prevent MOMP in the absence of stress (Figure 5CD for hetero-dimers between anti-apoptotic proteins, and BAK, Figure 5EF for free anti-apoptotic proteins).

To reconcile the predictions of the computational representation of the indirect activation model with the experimental fact that HeLa cells are robust to MOMP in the absence of stress, the implementation of the indirect model was looked at more carefully. Indeed, as the backward kinetics constant of BAK homo-oligomers (the speed with which the BAK oligomer dissociate) was used as a free parameter, it was investigated how the model predictions would change, and what biological information can be extracted from such an analysis. In particular, it was reasoned that an increase of this backward constant would lower the amount of BAK pores which would fall under the 10% threshold that was assumed to induce MOMP. Therefore, the backward reaction rate was increased in order to change the dissociation constant of BAK homo-oligomers (ratio between backward and forward constant) from 15 nM (Figures 5G,H,I, black solid line) to values of 30 nM (long dashed line, Figures 4B1-4B3), 60 nM (long/short-dashed), 120 nM (short-dashed), 240 nM (grained dotted) and 480 nM (fine dotted line). Indeed, it was found that BAK pore formation (Figure 4B) in HeLa cells was substantially reduced in absence of stress and no MOMP was observed when the dissociation constant was decreased to values below 120 nM. By similar means, HCT-116 and LoVo cells were predicted to undergo MOMP in the absence of stress for dissociation constants lower or equal than 120 nM (Figure 5H,I). In particular, in order to predict stability for LoVo cells in the absence of stress, an even higher backward reaction rate, and therefore an even weaker binding of BAK and BAX homo-oligomers of about 480 nM had to be assumed.

As seen in Figure 5J, the minimal required protein dose to induce MOMP in the indirect activation model (cross striped bar graph texture) was much higher than that calculated for the direct model (dotted bar graph texture). Strikingly, for HCT-116 cells the required protein dose was calculated to be 1.6 $\mu$M of BIM, PUMA and NOXA, and therefore twice as high as it would be required in the direct model. In conclusion, in order that the indirect model predicts stability in the absence of stress and MOMP in the presence of stress, a sufficiently weak oligomerisation and a sufficiently high protein dose is required.

*Individual protein profiles of colon cancer cell lines show excess of apoptotic effectors over anti-apoptotic BCL2 proteins*

To predict the kinetics of MOMP in individual cell lines, absolute levels of members of the BCL2 protein family are required. BAK, BAX, BCL-2, BCL-(X)L and MCL-1 protein levels were calculated by quantitative Western Blotting (n = 3 experiments) in HeLa cervical cancer cells and several colorectal cancer (CRC) cell lines. For HeLa cells, extracts were compared to purified proteins, and absolute protein levels were determined by densitometry. Levels of above proteins for Colo-205, DLD-1, HCT-116, HCT-116 *puma*$^{-/-}$, HCT-116 *p53*$^{-/-}$, HT-29 and LoVo CRC cells were obtained relative to HeLa cells by co-plotting the respective cell extracts. As result, HeLa cells were determined to express 1 $\mu$M BAK, 519 nM BAX, 239 nM BCL-2, 110 nM BCL-(X)L and 83 nM MCL-1. Concentrations for the colorectal cancer cell lines are depicted in Figure 6A.

Despite individual differences in the different cell lines, a striking pattern of protein expression was emerging which indicated that the total amount of apoptotic effectors was notably higher than the total amount of all quantified anti-apoptotic proteins. Most strikingly, HeLa cells showed more than three times higher levels of BAK and BAX (accumulated light and dark grey bars in the background) than total levels of anti-apoptotic proteins (black, dark grey and light grey bars for BCL-2, BCL- (X)L MCL-1 in the foreground). Likewise, HT-29 and Colo-205 cells revealed a twofold higher expression of total BAK and BAX compared to the sum of BCL-2, BCL-(X)L MCL-1 proteins. Only HCT-116 wild type and HCT-116 *p53*$^{-/-}$ and HCT-116 *puma*$^{-/-}$ mutant cell lines showed similar total levels of pro- and anti-apoptotic proteins. The inequality of pro- and anti-apoptotic proteins suggests that the mere inhibition of BAK and BAX by anti-apoptotic BCL2 proteins may not be sufficient to prevent MOMP in the absence of stress (unless BAK and BAX homo-oligomerisation is instable as modelled by a high dissociation constant in the previous section). However, the possibility cannot be excluded that other anti-apoptotic BCL2 family members (such as A1) or other inhibitors (such as VDAC2 in case of BAK) which were not quantified or other mechanisms that sequester BAK and BAX, or prevent BAX translocation, may prevent pore formation and MOMP.

*Model prediction of minimum stress necessary for MOMP correlates with amount of cell death in colorectal cancer cell lines*

Protein expression of the BCL2 family members may determine whether or not apoptosis is executed upon stress such as upon genotoxic stress following chemotherapeuic treatment.

To determine the sensitivity of different CRC cells to chemotherapeutic stimuli, Colo-205, HCT-116, HCT-116 *p53$^{-/-}$,* HT-29 and LoVo cells were subjected to 30$\mu$g/ml 5FU and 10$\mu$g/ml Oxaliplatin, a drug combination being the current standard of care in treatment of colorectal cancer. Using fluorescence-activated cell sorting (FACS), the fraction of cells that underwent cell death after 48 hr were quantified. As expected, cell death in the p53-mutated cell lines was low (40%, 23% and 20% for Colo-205, HCT-116 *p53$^{-/-}$,* and HT-29 cells, respectively) while a larger amount of cell death of 70% was observed in the p53-competent cell line HCT-116. Amount of cell death in the p53-competent cell line LoVo was 42%.

Whether the sensitivity of different CRC cell lines to genotoxic stress can be explained by their absolute BCL2 protein levels was investigated (Figure 6A). While protein levels may vary within a cell population, it was reasoned that a higher average amount of anti-apoptotic proteins in a population (the concentration of a "typical" cell of this population) would lead to a higher fraction of cells that survived genotoxic stimuli. Therefore, the accumulated levels of BCL-2, BCL-(X)L and MCL-1 which were determined by quantitative Western Blot were correlated to the survival rate of the FACS data from CRC cells as determined in the previous paragraph. A weak positive trend between amount of anti-apoptotic proteins and experimental amount of cell survival was observed. Linear regression, however, suggested only a very weak trend (small residual $R^2$ of 0.12). Likewise, neither Pearson's correlation (testing the linear dependency), nor Spearman rank correlation (testing a parameter free trend) confirmed a significant trend (Pearson coefficient = 0.35 with significance p = 0.50, Spearman rank coefficient = 0.31, p = 0.56; Figure 6B). Similarly, the total amount of BAK and BAX together did not reveal any significant trend.

Since it was not possible to identify any trend that relates the expression levels of BCL2 proteins to sensitivity of different cancer cell lines, it was investigated whether including the topology and kinetics of BCL2 interaction, as assumed by the model, would give a better explanation of the cancer cell sensitivity determined by the FACS data. Therefore, a model predictor was sought that, similar to the amount of anti-apoptotic proteins before, may be used to explain the amount of cell death and survival in cancer cells. To this end, it was reasoned that the activity of transcription, and therefore the rate of protein production, comes at a certain cost for the cell. Consequently, a higher amount of apoptotic stress due to 5FU/Oxaliplatin would induce a higher expression of BIM, PUMA and NOXA in p53 and PUMA competent cells, a higher expression of BIM and NOXA in HCT-116 *puma$^{-/-}$* cells (no PUMA expression assumed) and a higher expression of BIM in p53-mutated or deficient cells (no PUMA and NOXA expression assumed). It was therefore assumed that the minimal amount of protein dose in response to genotoxic stress that is required to induce MOMP in a typical cell of the population may be a good predictor for the apoptosis susceptibility of a cell population.

Results of the analysis are depicted in Figure 6C. Indeed, the minimum protein dose required to induce MOMP showed a good positive correlation with the amount of cell survival (the lower the required the dose, the more sensitive a cell population). This trend was indicated by a good linear regression ($R^2$ = 0.70) and by Pearson correlation confirming a significant trend (Pearson coefficient = 0.84 with significance p = 0.04).

*Model predicts patient tumour cells more susceptible to chemotherapeutically induced cell death than cells from matched normal tissue*

In chemotherapy, a therapeutic window is defined by the higher susceptibility of tumour cells to therapeutic stimuli compared to other cells of the same tissue (matched normal tissue). By using a set of chemotherapeutic drugs together with synthetic mimetics of BH3-only proteins, Certo *et. al.* (M. Certo, V. Del Gaizo Moore, M. Nishino, G. Wei, S. Korsmeyer, S. A. Armstrong, and A. Letai, 'Mitochondria primed by death signals determine cellular addiction to antiapoptotic BCL-2 family members', Cancer Cell, vol. 9, no. 5, pp. 351-365, May 2006) have recently shown that cancer cells may be indeed more susceptible to undergo drug induced MOMP than normal tissue. The model of the present invention was used to test their findings in a clinical setting.

Tumour and matched normal tissue samples of 27 stage II and stage III colorectal cancer (CRC) patients were obtained. Absolute protein expression levels of BAX, BAK, BCL-2, BCL-(X)L and MCL-1 were determined by comparing samples of tissue extracts and quantitative Western Blotting relative to extracts of HeLa cells as described above (Figure 7A and 7B for matched normal and tumour tissue, respectively). It was then reasoned that the amount of protein of BIM/ PUMA/NOXA which gets induced upon chemotherapeutically-induced stress is a surrogate of the amount of chemotherapeutical dose, reflecting the fact that protein production comes at certain costs for the cell, and, thus, is higher upon higher stress doses. The quantified protein data was used in the computational model of the present invention and the minimum protein dose of PUMA/NOXA/BIM induction was calculated as before that is necessary to induce MOMP in tumour and matched normal tissue of each individual patient.

Strikingly, the computational model of the present invention predicted that, in average over all patients, cells from tumour tissue required a statistically significantly lower protein dose of BIM/PUMA/NOXA (median value of 410 nM) than cells from matched normal tissues (median of 818 nM; p = 0.02, Wilcoxon-signed rank test; Figure 7C). These results suggest that a lower dosage of 5FU/oxaliplatin-based chemotherapy is required to induce tumour cell death in these CRC patients. This lower dose to induce cancer cell death was found in the majority (21 out of 27) of patients as illustrated by the lines between data points that indicate correspondence between healthy matched normal and tumour tissue of an individual patient (Figure 7C). It should be noted that the required protein dose to induce cell death in healthy tissue showed a much higher variability than the dose required to induce cancer cell death (indicated by the whiskers of both box-plots), suggesting that the optimal dose must be decided on a patient individual level.

The available information on the 4-years disease free survival of each patient was used in the computational model of the present invention and the protein dose for each patient group was calculated separately. The results indeed indicated that patients with 4-years disease free survival required a significantly smaller protein dose of a median of 191 nM BIM/PUMA/NOXA, while patients with disease recurrence within 4-years required a median dose of 620 nM to kill their tumour cells (Figure 7D; p = 0.006, Wilcoxon-signed rank test). In addition, a high variability in the required minimal protein dose was calculated for the latter group of patients, suggesting again that responses to chemotherapeutic doses are highly patient individual.

*Systems modelling can be a tool for patient individual dosage decisions and for personalised treatment options*

In the previous paragraph the computational model of the present invention predicted that over all of the 27 CRC patients, tumour tissue was more sensitive to chemotherapy than healthy matched normal tissue and those patients whose cancer cells were more sensitive to chemotherapy than cancer cells of other patients had favourable clinical outcome. However, modelling also indicated that the predicted protein dose that was necessary to induce MOMP (and thus the necessary dose of chemotherapy) varied considerably between patients, suggesting that treatment decisions need to be analysed on an individual patient level. Analysis of the data presented in Figure 7 for the predicted therapeutic window, defined as the difference in the predicted protein dose between healthy and tumour tissue, for each patient individually was performed. As suspected, a high variability in the size of the therapeutic window was found between patients as indicated by the arrow lengths in Figure 8A.

BH3-mimetics are drugs that sensitise cancer cells to apoptosis by mimicking the BH3-domain of BH3-only proteins and are currently in clinical trial for adjuvant chemotherapy. The synthetic drug ABT-737 has been shown to induce apoptosis by binding with strong affinity to BCL-2 and BCL-(X)L leading to a de-repression of BH3-only activators. By similar means, the lipid Gossypol that naturally occurs in cotton seed sensitises cell to apoptosis by its binding to the anti-apoptotic proteins BCL-2 and MCL-1. As BH3-mimetics are currently under clinical trial for their use as co-treatments to standard chemotherapy, it was considered whether the computational model of the present invention could predict whether or not and to what amount both mimetics would lower the necessary chemotherapeutic dose for tumour tissue of individual patients.

The analysis from the previous section was revisited with the inclusion of the molecular interactions of Gossypol or ABT-737 in the computational model. Due to mimetic administration of either gossypol or ABT-737 it was modelled that 1 $\mu$M or 2.5 $\mu$M were taken up by the cells over 12hr. Subsequently, classical 5FU/oxaliplatin based chemotherapy was applied and again the protein dose of BIM/PUMA/NOXA that was necessary for inducing MOMP in the tumour tissue of individual patients was calculated. Results indicate that the median of all patients of the minimum protein dose that is required for MOMP in tumour tissue decreased. In particular this median was reduced from 410 nM to 180 nM when a dose of 1 $\mu$M ABT-737 was modelled to be present and to 150 nM when 2.5 $\mu$M ABT-737 was assumed (Figure 8B). Similarly, assuming Gossypol instead of ABT-737 reduced the necessary median protein dose to 180 nM and 160 nM for both concentrations. It was noted that increasing the mimetic dose also decreased patient variability of the necessary protein dose. Across all the patients the reduction in the necessary protein dose, and therefore in the assumed dosage in 5FU/oxaliplatin based chemotherapy, was similar for co-treatments with ABT-737 and gossypol.; However in some patients, such as patient 8028 (Figure 8B inset), the minimum dose of required genotoxic chemotherapy was substantially more reduced by ABT-737 than by Gossypol. Therefore, the computational model of the present invention allows assessing for optimal drugs that sensitise cells to apoptosis by mimicking pro-apoptotic BCL2 proteins.

Table 1

| A | protein and protein complex degradation | |
|---|---|---|
| $C \xrightarrow{k_{\deg}}$ | to | $\dfrac{d[C]}{dt} = -k_{\deg} * [C]$<br><br>whereby $\lim\limits_{t \to \infty}[C] = \dfrac{k_{prod}}{k_{\deg}}$ |
| | | |
| B | protein turnover | |
| $\xrightarrow{k_{prod}} C$<br>$k_{\deg}$ | | $\dfrac{d[C]}{dt} = k_{prod} - k_{\deg} * [C]$ |
| | | |
| C | protein translocation or reaction | |
| $R \xrightarrow{k} P$ | to | $\dfrac{d[R]}{dt} = -k * [R]$<br><br>$\dfrac{d[P]}{dt} = k * [R]$ |
| | | |
| D | irreversible protein reaction | |
| $R_1 + R_2 \xrightarrow{k} P$ | to | $\dfrac{d[R_1]}{dt} = \dfrac{d[R_2]}{dt} = -k * [R_1] * [R_2]$<br><br>$\dfrac{d[P]}{dt} = k * [R_1] * [R_2]$ |
| E | reversible protein reaction (association and dissociation) | |
| $R_1 + R_2 \xleftrightarrow[k_{backward}]{k_{forward}} R_1 \sim R_2$ | to | $\dfrac{d[R_1]}{dt} = \dfrac{d[R_2]}{dt} = k_{backward} * [R_1 \sim R_2] - k_{forward} * [R_1] * [R_2]$<br><br>$\dfrac{d[R_1 \sim R_2]}{dt} = k_{forward} * [R_1] * [R_2] - k_{backward} * [R_1 \sim R_2]$<br><br>whereby $K_D = \dfrac{k_{backward}}{k_{forward}}$ |

## Table 2

| The degradation constant $k_{deg}$ was determined by the following equation: $k_{deg} = \ln(2)/(60 \cdot t_{1/2})$ | | | |
|---|---|---|---|
| **Biochemical reaction** | | $k_{deg}$ [s$^{-1}$] | $t_{1/2}$ [min] |
| Bcl-2 | → | 3.85E-05 | 300 |
| Bcl-(X)L | → | 3.85E-05 | 300 |
| Bcl-W | → | 3.85E-05 | 300 |
| Mcl-1 | → | 2.57E-04 | 45 |
| Mcl-1 | → | 6.80E-04 | 17 |
| Bim | → | 4.81E-05 | 240 |
| tBid | → | 1.54E-04 | 75 |
| Puma | → | 5.66E-05 | 204 |
| Noxa | → | 1.93E-04 | 60 |
| Bcl-2 ~ Bim | → | 1.54E-05 | 75 |
| Bcl-(X)L ~ Bim | → | 1.54E-05 | 75 |
| Bcl-W ~ Bim | → | 1.54E-05 | 75 |
| Mcl-1 ~ Bim | → | 7.70E-05 | 150 |
| **Biochemical reaction** | | $k_{deg}$ [s$^{-1}$] | $t_{1/2}$ [min] |
| Bcl-2 ~ tBid | → | 1.54E-05 | 75 |
| Bcl-(X)L ~ tBid | → | 1.54E-05 | 75 |
| Bcl-W ~ tBid | → | 1.54E-05 | 75 |
| Mcl-1 ~ tBid | → | 1.54E-05 | 75 |
| Bcl-2 ~ Puma | → | 1.54E-05 | 75 |
| Bcl-(X)L ~ Puma | → | 1.54E-05 | 75 |
| Bcl-W ~ Puma | → | 1.54E-05 | 75 |
| Mcl-1 ~ Puma | → | 7.70E-05 | 150 |
| Bcl-2 ~ Noxa | → | 1.54E-05 | 75 |
| Bcl-(X)L ~ Noxa | → | 1.54E-05 | 75 |
| Bcl-W ~ Noxa | → | 1.54E-05 | 75 |
| Mcl-1 ~ Noxa | → | 2.57E-04 | 45 |

## Table 3

| The forward constant $k_{forward}$, except for that obtained from Chen *et. al.,* were determined from the backward constant $k_{backward}$ and dissociation constant $K_D$, by using the following equation: $k_{forward} = k_{backward} / K_D$ | | | | | |
|---|---|---|---|---|---|
| **A** | **Biochemical reaction** | | $k_{forward}$ [nM$^{-1}$s$^{-1}$] | $k_{backward}$ [s$^{-1}$] | $K_D$ [nM] |
| Bcl-2 + Bim | ↔ | Bcl-2 ~ Bim | 3.00E-05 | 1.40E-04 | 4.5 |
| Bcl-2 + tBid | ↔ | Bcl-2 ~ tBid | 3.50E-05 | 1.40E-04 | 4.0 |
| Bcl-2 + Puma | ↔ | Bcl-2 ~ Puma | 7.78E-06 | 1.40E-04 | 18.0 |

(continued)

| A | Biochemical reaction | | | $k_{forward}$ [nM$^{-1}$s$^{-1}$] | $k_{backward}$ [s$^{-1}$] | $K_D$ [nM] |
|---|---|---|---|---|---|---|
| Bcl-2 + Noxa | ↔ | | Bcl-2 ~ Noxa | 7.29E-07 | 1.40E-04 | 192.0 |
| Bcl-2 + Bak | ↔ | | Bcl-2 ~ Bak | 1.40E-08 | 1.40E-04 | 10,000 |
| Bcl-2 + Bax | ↔ | | Bcl-2 ~ Bax | 9.33E-06 | 1.40E-04 | 15.0 |

| B | Biochemical reaction | | | $k_{forward}$ [nM$^{-1}$s$^{-1}$] | $k_{backward}$ [s$^{-1}$] | $K_D$ [nM] |
|---|---|---|---|---|---|---|
| Bcl-(X)L + Bim | ↔ | | Bcl-(X)L ~ Bim | 5.50E-04 | 4.40E-04 | 0.8 |
| Bcl-(X)L + tBid | ↔ | | Bcl-(X)L ~ tBid | 1.62E-05 | 4.40E-04 | 27.2 |
| Bcl-(X)L + Puma ↔ | | | Bcl-(X)L ~ Puma | 8.63E-05 | 4.40E-04 | 5.1 |
| Bcl-(X)L + Noxa ↔ | | | Bcl-(X)L ~ Noxa | 4.40E-08 | 4.40E-04 | 10,000 |
| Bcl-(X)L + Bak | ↔ | | Bcl-(X)L ~ Bak | 5.50E-06 | 4.40E-04 | 80.0 |
| Bcl-(X)L + Bax | ↔ | | Bcl-(X)L ~ Bax | 5.18E-07 | 4.40E-04 | 850.0 |

| C | Biochemical reaction | | | $k_{forward}$ [nM$^{-1}$s$^{-1}$] | $k_{backward}$ [s$^{-1}$] | $K_D$ [nM] |
|---|---|---|---|---|---|---|
| Mcl-1 + Bim | ↔ | | Mcl-1 ~ Bim | 1.30E-03 | 2.60E-04 | 0.2 |
| Mcl-1 + tBid | ↔ | | Mcl-1 ~ tBid | 2.63E-05 | 2.60E-04 | 9.9 |
| Mcl-1 + Puma | ↔ | | Mcl-1 ~ Puma | 1.37E-04 | 2.60E-04 | 1.9 |
| Mcl-1 + Noxa | ↔ | | Mcl-1 ~ Noxa | 6.58E-06 | 2.60E-04 | 39.9 |
| Mcl-1 + Bak | ↔ | | Mcl-1 ~ Bak | 3.25E-05 | 2.60E-04 | 8.0 |
| Mcl-1 + Bax | ↔ | | Mcl-1 ~ Bax | 2.60E-08 | 2.60E-04 | 10,000 |

| D | | Biochemical reaction | | $k_{forward}$ [nM$^{-1}$s$^{-1}$] | $k_{backward}$ [s$^{-1}$] | $K_D$ [nM] |
|---|---|---|---|---|---|---|
| Bcl-W + Bim | ↔ | | Bcl-W ~ Bim | 1.17E-04 | 2.70E-03 | 23.0 |
| Bcl-W + tBid | ↔ | | Bcl-W ~ tBid | 1.42E-04 | 2.70E-03 | 19.0 |
| Bcl-W + Puma | ↔ | | Bcl-W ~ Puma | 1.08E-04 | 2.70E-03 | 25.0 |
| Bcl-W + Noxa | ↔ | | Bcl-W ~ Noxa | 1.08E-06 | 2.70E-03 | 2,500.0 |
| Bcl-W + Bak | ↔ | | Bcl-W ~ Bak | 9.64E-06 | 2.70E-03 | 280.0 |
| Bcl-W + Bax | ↔ | | Bcl-W ~ Bax | 1. 17E-04 | 2.70E-03 | 23.0 |

## Table 4

| A | Biochemical reaction | | | $k_{forward}$ [nM$^{-1}$s$^{-1}$] | $k_{backward}$ [s$^{-1}$] | $K_D$ [nM] |
|---|---|---|---|---|---|---|
| Bax$_c$ + Activator | ↔ | | Bax$_c$ ~ Activator | 2.57E-06 | 2.57E-04 | 100.0 |
| Bak$_m$ + Activator | ↔ | | Bak$_m$ ~ Activator | 2.57E-06 | 2.57E-04 | 100.0 |

(continued)

| B | | | Biochemical reaction | k [s$^{-1}$] | t$_{1/2}$ [min] |
|---|---|---|---|---|---|
| Bak$_m$ ~ Activator | $\rightarrow$ | | Bak*$_m$ + Activator | 1.16E-01 | 0.1 |
| Bax$_c$ ~ Activator | $\rightarrow$ | | Bax*$_c$ + Activator | 1.16E-01 | 0.1 |

| C | | | Biochemical reaction | k [s$^{-1}$] | t$_{1/2}$ [min] |
|---|---|---|---|---|---|
| Bax*$_c$ | $\rightarrow$ | | Bax*$_m$ | 1.16E-01 | 0.1 |

| D | | | Biochemical reaction | k$_{forward}$ [nM$^{-1}$s$^{-1}$] | k$_{backward}$ [s$^{-1}$] | K$_D$ [nM] |
|---|---|---|---|---|---|---|
| Bak$_m$ + VDAC2 $\leftrightarrow$ | $\leftrightarrow$ | | Bak$_m$ ~ VDAC2 | 2.31E-06 | 2.31E-03 | 1,000.0 |

**Table 5**

| Biochemical reaction | | | k$_{forward}$ [nM$^{-1}$s$^{-1}$] | k$_{backward}$ [s$^{-1}$] | K$_D$ [nM] |
|---|---|---|---|---|---|
| Effector*$_m$ + Effector*$_m$ | $\leftrightarrow$ | Effector$^2_m$ | 1.28E-05 | 1.93E-04 | 15 |
| Effector$^2_m$ + Effector$^2_m$ | $\leftrightarrow$ | Effector$^4_m$ | 1.28E-05 | 1.93E-04 | 15 |
| Effector$^2_m$ + Effector$^4_m$ | $\leftrightarrow$ | Effector$^6_m$ | 1.28E-05 | 1.93E-04 | 15 |
| Effector$^2_m$ + Effector$^6_m$ | $\leftrightarrow$ | Effector$^8_m$ | 1.28E-05 | 1.93E-04 | 15 |
| Effector$^4_m$ + Effector$^4_m$ | $\leftrightarrow$ | Effector$^8_m$ | 1.28E-05 | 1.93E-04 | 15 |
| Effector$^4_m$ + Effector$^7_m$ | $\leftrightarrow$ | Effector$^{10}_m$ | 1.28E-05 | 1.93E-04 | 15 |
| Effector$^2_m$ + Effector$^8_m$ | $\leftrightarrow$ | Effector$^{10}_m$ | 1.28E-05 | 1.93E-04 | 15 |
| Effector$^6_m$ + Effector$^6_m$ | $\leftrightarrow$ | Effector$^{12}_m$ | 1.28E-05 | 1.93E-04 | 15 |
| Effector$^4_m$ + Effector$^8_m$ | $\leftrightarrow$ | Effector$^{12}_m$ | 1.28E-05 | 1.93E-04 | 15 |
| Effector$^2_m$ + Effector$^{10}_m$ | $\leftrightarrow$ | Effector$^{12}_m$ | 1.28E-05 | 1.93E-04 | 15 |

**Table 6**

| 1 hour | | | | | | |
|---|---|---|---|---|---|---|
| A | | Biochemical reaction | | k$_{forward}$ [nM$^{-1}$s$^{-1}$] | k$_{backward}$ [s$^{-1}$] | K$_D$ [nM] |
| Gossypol + Bcl-2 | $\leftrightarrow$ | | Gossypol ~ Bcl-2 | 5.50E-06 | 1.93E-04 | 35.0 |
| Gossypol + Bcl-(X)L | $\leftrightarrow$ | | Gossypol ~ Bcl-(X)L | 2.92E-07 | 1.93E-04 | 660.0 |
| Gossypol + Mcl-1 | $\leftrightarrow$ | | Gossypol ~ Mcl-1 | 7.70E-06 | 1.93E-04 | 25.0 |
| Abt-737 + Bcl-2 | $\leftrightarrow$ | | Abt-737 ~ Bcl-2 | 1.93E-04 | 1.93E-04 | 1.0 |
| Abt-737 + Bcl-(X)L | $\leftrightarrow$ | | Abt-737 ~ Bcl-(X)L | 3.85E-04 | 1.93E-04 | 0.5 |
| Abt-737 + Mcl-1 | $\leftrightarrow$ | | Abt-737 ~ Mcl-1 | 1.93E-07 | 1.93E-04 | 1,000.0 |

(continued)

| B | Biochemical reaction | | $k_{deg}$ [s$^{-1}$] | $t_{1/2}$ [min] |
|---|---|---|---|---|
| Gossypol | $\rightarrow$ | | 2.41E-05 | 480 |
| Gossypol $\sim$ Bcl-2 | $\rightarrow$ | | 1.54E-04 | 75 |
| Gossypol $\sim$ Bcl-(X)L | $\rightarrow$ | | 1.54E-04 | 75 |
| Gossypol $\sim$ Mcl-1 | $\rightarrow$ | | 7.70E-05 | 150 |
| Abt-737 | $\rightarrow$ | | 9.63E-05 | 120 |
| Abt-737 $\sim$ Bcl-2 | $\rightarrow$ | | 1.54E-04 | 75 |
| Abt-737 $\sim$ Bcl-(X)L | $\rightarrow$ | | 1.54E-04 | 75 |
| Abt-737 $\sim$ Mcl-1 | $\rightarrow$ | | 7.70E-05 | 150 |

**Table 7**

| A | Bak [nM] | Bax [nM] | Bcl-2 [nM] | Bcl-(X)L [nM] | MCL-1 [nM] | |
|---|---|---|---|---|---|---|
| HeLa | 1,009.24 | 519.41 | 239.07 | 109.98 | 83.38 | |
| COLO-205 | 467.24 | 959.58 | 84.30 | 484.40 | 5.20 | |
| DLD-1 | 779.22 | 409.67 | 22.17 | 199.30 | 11.64 | |
| HCT-116 | 676.65 | 317.56 | 230.64 | 604.41 | 25.26 | |
| HCT-116 p53$^{-/-}$ | 559.79 | 278.28 | 342.07 | 603.95 | 23.27 | |
| HCT-116 puma$^{-/-}$ | 338.95 | 320.94 | 129.70 | 456.29 | 6.90 | |
| HT-29 | 1,306.89 | 972.29 | 46.56 | 994.13 | 3.81 | |
| LoVo | 1,932.36 | 0.00 | 653.24 | 653.73 | 37.03 | |
| B | Bak [nM] | Bax [nM] | Bcl-2 [nM] | Bcl-(X)L [nM] | Mcl-1 [nM] | Bcl-W [nM] |
| HeLa | 1,009.24 | 519.41 | 239.07 | 109.98 | 83.38 | 0.00 |
| MZ18 | 2,405.46 | 768.61 | 593.87 | 70.92 | 28.84 | 4.39 |
| MZ51 | 2,054.25 | 1,539.02 | 667.99 | 130.07 | 12.89 | 1.13 |
| MZ256 | 1,775.90 | 795.37 | 1,225.07 | 88.80 | 37.35 | 1.78 |
| MZ294 | 1,734.25 | 1,135.28 | 567.82 | 143.39 | 14.56 | 0.72 |
| MZ304 | 4,939.34 | 277.19 | 392.61 | 93.64 | 60.39 | 1.61 |
| MZ327 | 2,696.81 | 586.09 | 647.55 | 106.33 | 33.14 | 1.10 |
| U87 | 4,000.17 | 1,021.48 | 585.66 | 161.19 | 60.91 | 3.60 |
| U251 | 1,036.79 | 524.32 | 439.93 | 167.57 | 29.58 | 2.40 |
| U343 | 1,644.64 | 3,120.45 | 1,662.55 | 224.87 | 26.60 | 2.95 |
| U373 | 1,315.99 | 382.66 | 412.56 | 238.56 | 20.79 | 2.85 |
| A172 | 1,983.25 | 1,175.37 | 111.37 | 161.48 | 23.79 | 1.18 |

In conclusion, to date, no clinical tool allows a fast, cheap and exact determination of the patient-specific, quantitative proteomic fingerprints of malignant tissue and studies protein-protein interactions based on these fingerprints to predict treatment response in individual patients. Therefore, the computational model of the present invention can be applied for clinical decision making. In addition, the computational model may aide in the decision whether or not compounds with certain molecular interaction characteristics may go into clinical trial. The computational model of the present invention allows a patient specific investigation of the treatment dose and may point to the most suitable co-treatments that are applied in support of classical 5FU/Oxaliplatin based chemotherapy.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A computer-implemented method for predicting quantitatively whether an adjuvant or neoadjuvant chemotherapeutic treatment will be or is being successful in treating an individual suffering from cancer, the method comprising the steps of:

   assaying a cancerous biological sample and a normal biological sample from the individual to determine the concentration of two or more BCL-2 family members in each sample;
   inputting the concentration value for at least two BCL-2 family members of each sample into a non-linear protein-protein network computational model and adapted to calculate quantitative protein profiles over time from quantitative molecular interaction data and assess the mitochondrial outer membrane permeabilisation apoptosis pathway invoked by chemotherapeutically-induced stress;
   processing said concentration values using said computational model to determine the interaction of pro-apoptotic and pro-survival BCL-2 family members invoked after chemotherapeutic-induced stress in the sample; and
   outputting a quantitative prediction value of the likelihood of treatment success using the adjuvant or neoadjuvant chemotherapeutic treatment.

2. A computer-implemented method according to Claim 1, wherein the method comprises the further step of outputting a quantitative, dose-dependent prediction value of the likelihood of treatment success using novel apoptosis sensitizers.

3. A computer-implemented method according to Claim 1 further comprising the step of estimating a minimal quantitative dose value of a chemotherapeutic required to induce the mitochondrial outer membrane permeabilisation apoptosis pathway in the sample, such that any cancer cell is killed while preserving healthy cells of the individual suffering from cancer.

4. A computer-implemented method according to Claim 3, wherein the quantitative minimal dose is estimated based on the concentration values of the BCL-2 family members required to induce the process of mitochondrial outer membrane permeabilisation in the cancer cell.

5. A computer-implemented method according to any one of Claims 1 to 4, wherein the concentration values inputted into the computational model are processed to provide an apoptosis status, which is correlated with a known response to provide said quantitative, dose-dependent prediction value to predict the individual's response to treatment and/or the minimal dose necessary to kill cancer cells.

6. A computer-implemented method according to any one of the preceding claims, wherein said processing step calculates an activation profile over time of pro-apoptotic effector BCL-2 family member proteins and compares the result with known results to quantitatively predict the individual's response to treatment and/or the minimal dose necessary to kill cancer cells.

7. A computer-implemented method according to any preceding claim, wherein the BCL-2 family members are selected from BAK, BAX, BCL-2, BCL-(X)L, BCL-(X)S, BCL-W, A1, MCL-1, BIM, BID, BAD, PUMA and NOXA.

8. A computer implemented method according to Claim 6 or Claim 7, wherein the pro-apoptotic effector BCL-2 proteins are BAK and BAX.

9. A computer-implemented method according to any preceding claims, wherein the concentration value for each of the at least two BCL-2 family member is representative of protein levels for the sample.

10. A computer-implemented method according to any one of the preceding claims, wherein the biological sample is selected from the group comprising a blood sample, especially blood serum or plasma, cerebrospinal fluid, saliva, urine, lymphatic fluid, or cell or tissue extracts, or a biopsy or tissue biopsy.

11. A computer-implemented method according to any preceding claim wherein the step of determining the concentration of the BCL-2 family members comprises obtaining protein profiles by any one or more of: tissue microarray immunostaining, immunohistochemistry, reverse phase protein array analysis, or quantitative Western blot.

12. A computer-implemented method according to any preceding claim wherein the cancer is selected from the group consisting of myeloma, prostate cancer, glioblastoma, lymphoma, fibrosarcoma; myxosarcoma; liposarcoma; chondrosarcom; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias.

13. A computer-implemented system for predicting quantitatively whether an adjuvant or neoadjuvant chemotherapeutic treatment will be or is being successful in treating an individual suffering from cancer, the system comprising:

    means for assaying a cancerous biological sample and a normal biological sample from the individual to determine the concentration of two or more BCL-2 family members in each sample;
    means for inputting the concentration value for at least two BCL-2 family members of each sample into a nonlinear protein-protein network computational model and adapted to calculate quantitative protein profiles over time from quantitative molecular interaction data and assess the mitochondrial outer membrane permeabilisation apoptosis pathway invoked by chemotherapeutically-induced stress;
    means for processing said concentration values using said computational model to quantitatively determine the interaction of pro-apoptotic and pro-survival BCL-2 family members invoked after chemotherapeutic-induced stress in the sample; and
    means for outputting a quantitative prediction value of the likelihood of treatment success using the adjuvant or neoadjuvant chemotherapeutic treatment.

14. A computer-implemented system according to Claim 13, wherein said processing step calculates an activation profile over time of pro-apoptotic effector BCL-2 family member proteins and compares the result with known results to quantitatively predict the individual's response to treatment and/or the minimal dose necessary to kill cancer cells.

15. A computer-implemented system according to any one of Claims 12 to 14, wherein the BCL-2 family members are selected from BAK, BAX, BCL-2, BCL-(X)L, BCL-(X)S, BCL-W, A1, MCL-1, BIM, BID, BAD, PUMA and NOXA.

Figure 1

Figure 2 A-D

**Figure 2 E1-F3**

Figure 3

Figure 4A

Figure 4B

28

Figure 5

Figure 6

Figure 7

Figure 8

Europäisches
Patentamt

European
Patent Office

Office européen
des brevets

# EUROPEAN SEARCH REPORT

Application Number

EP 12 16 6187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAIN H. V. ET AL: "The Molecular Basis of Synergism between Carboplatin and ABT-737 Therapy Targeting Ovarian Carcinomas", CANCER RESEARCH, vol. 71, no. 3, 1 February 2011 (2011-02-01), pages 705-715, XP055048765, ISSN: 0008-5472, DOI: 10.1158/0008-5472.CAN-10-3174 * the whole document * | 1-15 | INV. G06F19/18 ADD. G06F19/12 |
| X | SJÖSTRÖM J. ET AL: "The predictive value of bcl-2, bax, bcl-xL, bag-1, fas, and fasL for chemotherapy response in advanced breast cancer", CLINICAL CANCER RESEARCH, vol. 8, no. 3, 1 January 2002 (2002-01-01), pages 811-816, XP055051548, ISSN: 1078-0432 * abstract * * page 811, right-hand column, line 21 - page 813, right-hand column, line 15 * | 1-15 | |
| A | WELLER M.: "Predicting response to cancer chemotherapy: the role of p53", CELL AND TISSUE RESEARCH, vol. 292, no. 3, 20 May 1998 (1998-05-20), pages 435-445, XP055051549, ISSN: 0302-766X, DOI: 10.1007/s004410051072 * the whole document * | 1-15 | TECHNICAL FIELDS SEARCHED (IPC) G06F |

-/--

The present search report has been drawn up for all claims

| Place of search | Date of completion of the search | Examiner |
|---|---|---|
| The Hague | 31 January 2013 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 12 16 6187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AYERS M. ET AL: "GENE EXPRESSION PROFILES PREDICT COMPLETE PATHOLOGIC RESPONSE TO NEOADJUVANT PACLITAXEL AND FLUOROURACIL, DOXORUBICIN, AND CYCLOPHOSPHAMIDE CHEMOTHERAPY IN BREAST CANCER", JOURNAL OF CLINICAL ONCOLOGY, AMERICAN SOCIETY OF CLINICAL ONCOLOGY, US, vol. 22, no. 12, 15 June 2004 (2004-06-15) , pages 2284-2293, XP008043042, ISSN: 0732-183X, DOI: 10.1200/JCO.2004.05.166 * the whole document * | 1-15 | |
| A | FANEYTE I. F. ET AL: "Breast cancer response to neoadjuvant chemotherapy: predictive markers and relation with outcome", BRITISH JOURNAL OF CANCER, vol. 88, no. 3, 10 February 2003 (2003-02-10), pages 406-412, XP055049959, ISSN: 0007-0920, DOI: 10.1038/sj.bjc.6600749 * the whole document * | 1-15 | |
| A | IGNEY F. H. ET AL: "Death and anti-death: Tumour resistance to apoptosis", NATURE REVIEWS. CANCER, NATUR PUBLISHING GROUP, LONDON, GB, vol. 2, no. 4, 20 April 2002 (2002-04-20), pages 277-288, XP002251126, ISSN: 1474-175X, DOI: 10.1038/NRC776 * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 31 January 2013 | Godzina, Przemyslaw |

EPO FORM 1503 03.82 (P04C01)

Europäisches
Patentamt
European
Patent Office
Office européen
des brevets

**EUROPEAN SEARCH REPORT**

Application Number

EP 12 16 6187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAFFNEY E. A.: "The application of mathematical modelling to aspects of adjuvant chemotherapy scheduling", JOURNAL OF MATHEMATICAL BIOLOGY, vol. 48, no. 4, 1 April 2004 (2004-04-01), pages 375-422, XP055050488, ISSN: 0303-6812, DOI: 10.1007/s00285-003-0246-2 * the whole document * | 1-15 | |

-----

TECHNICAL FIELDS
SEARCHED        (IPC)

The present search report has been drawn up for all claims

| Place of search | Date of completion of the search | Examiner |
|---|---|---|
| The Hague | 31 January 2013 | Godzina, Przemyslaw |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. ZHANG ; P. BRAZHNIK ; J. J. TYSON.** Computational analysis of dynamical responses to the intrinsic pathway of programmed cell death. *Biophys J,* 2009, vol. 97, 415-34
- **L. CHEN ; S. N. WILLIS ; A. WEI ; B. J. SMITH ; J. I. FLETCHER ; M. G. HINDS ; P. M. COLMAN ; C. L. DAY ; J. M. ADAMS ; D. C. S. HUANG.** Differential targeting of prosurvival Bcl-2 proteins by their BH3-only ligands allows complementary apoptotic function. *Mol. Cell,* February 2005, vol. 17 (3), 393-403
- **G. LESSENE ; P. E. CZABOTAR ; P. M. COLMAN.** BCL-2 family antagonists for cancer therapy. *Nat Rev Drug Discov,* December 2008, vol. 7 (12), 989-1000
- **F. LLAMBI ; T. MOLDOVEANU ; S. W. G. TAIT ; L. BOUCHIER-HAYES ; J. TEMIROV ; L. L. MCCORMICK ; C. P. DILLON ; D. R. GREEN.** A unified model of mammalian BCL-2 protein family interactions at the mitochondria. *Mol. Cell,* November 2011, vol. 44 (4), 517-531
- **CONCANNON CG ; KOEHLER BF ; REIMERTZ C ; MURPHY BM ; BONNER C ; THUROW N ; WARD MW ; VILLUNGER A ; STRASSER A ; KÖGEL D.** Apoptosis induced by proteasome inhibition in cancer cells: predominant role of the p53/PUMA pathway. *Oncogene,* 15 March 2007, vol. 26 (12), 1681-92
- **M. REHM ; H. J. HUBER ; H. DUSSMANN ; J. H. M. PREHN.** Systems analysis of effector caspase activation and its control by X-linked inhibitor of apoptosis protein. *EMBO J.,* September 2006, vol. 25 (18), 4338-4349
- **A. LETAI ; M. C. BASSIK ; L. D. WALENSKY ; M. D. SORCINELLI ; S. WEILER ; S. J. KORSMEYER.** Distinct BH3 domains either sensitize or activate mitochondrial apoptosis, serving as prototype cancer therapeutics. *Cancer Cell,* September 2002, vol. 2 (3), 183-192
- **D. C. HUANG ; A. STRASSER.** BH3-Only proteins-essential initiators of apoptotic cell death. *Cell,* December 2000, vol. 103 (6), 839-842
- **M. CERTO ; V. DEL GAIZO MOORE ; M. NISHINO ; G. WEI ; S. KORSMEYER ; S. A. ARMSTRONG ; A. LETAI.** Mitochondria primed by death signals determine cellular addiction to antiapoptotic BCL-2 family members. *Cancer Cell,* May 2006, vol. 9 (5), 351-365